# EUROPEAN PATENT SPECIFICATION

(11) **EP 0 361 768 B1**
(45) Date of publication and mention of the grant of the patent: **08.11.1995**
(21) Application number: 89309532.3
(22) Date of filing: 19.09.1989
(51) Int. Cl.: C12Q 1/68, G01N 33/535

(54) **Enzyme-nucleic acid hydrazone and hydrazide conjugate compositions, probes and methods**
Enzym-Nukleinsäurehydrazon und Enzym-Nukleinsäurehydrazid-Konjugat-Zusammensetzung, Sonden und Verfahren
Compositions à base de conjugués d'une enzyme et d'un hydrazone ou hydrazide d'un acide nucléique, sondes et méthodes

(30) Priority: 26.09.1988 US 249766
(43) Date of publication of application: 04.04.1990
(60) Divisional application: 95105491.5
(73) Proprietor: SISKA DIAGNOSTICS,INC., La Jolla, CA 92037 (US)
(72) Inventor: Ghosh, Soumitra Shankar, San Diego, CA 92121 (US)
(74) Representative: Allard, Susan Joyce

(56) References cited:
- EP-A- 0 119 448
- EP-A- 0 209 996
- EP-A- 0 212 546
- WO-A-86/00074
- US-A- 4 537 718

## Description

The present invention concerns novel nucleic acid probes, wherein the label is a catalytically active enzyme joined covalently to the nucleic acid, novel nucleic acid intermediates for making such probes, and novel methods of making and using the probes.

Nucleic acid probes have multiple uses in probing and identifying DNA and RNA analytes in samples of biological material. Applications of nucleic acid probe hybridization assays include the detection of genetic diseases, diagnosis of infectious diseases, detection of foodstuff contamination, detection of desired clones among clones derived from a transformed culture, determination of the nature and extent of mutations in cloned DNA fragments, and the construction of maps of DNA molecules and chromosomes.

The most commonly employed method for labeling DNA and RNA molecules, such as synthetic oligonucleotides, so that they can be detected and, therefore, used as probes in nucleic acid probe hybridization assays, is enzymatic or chemical radiolabeling with a radioisotope, such as ³²P, ³H, or ³⁵S. Radioactively labeled nucleic acid probes, including oligonucleotide probes of fewer than about 100 bases, can provide high sensitivity and specificity in assays. However, such labeling is undesirable and inefficient, because radioactive nucleic acid molecules have short shelf-lives, and, during labeling, in hybridization assay procedures, and for disposal, require special equipment and precautions to minimize exposure of personnel and the environment to hazardous radioactivity. Widespread and routine use of nucleic acid probes in clinical, diagnostic, and other applications will require non-radioactive labels which are simple to use and provide sensitivity and specificity at least comparable to that provided by radioactively labeled probes.

Commonly employed, non-radioactive nucleic acid probe detection systems involve predominantly indirect methods, wherein the nucleic acid probe that hybridizes to target DNA or RNA analyte in a sample is covalently joined, through a linking moiety or "linker," with biotin or a low molecular weight hapten as label and wherein signal for detecting the probe is generated using a protein complex that is bound (non-covalently) to the biotin or hapten. For example, nucleic acids conjugated to biotin, through covalent linkages to a terminal nucleotide (e.g., through a 5'-carbon of the 5'-nucleotide) or at one or more nucleoside bases, are known. See, e.g., Chu and Orgel, DNA 4, 327-331 (1985); Ward et al., United States Patent No. 4,711,955. Such a biotinylated nucleic acid probe is detected, after hybridization of probe to its target and washing of excess unhybridized probe from the assay system, by adding to the system a complex of avidin or streptavidin, which are proteins which have very high affinities for biotin, with an enzyme, such as horseradish peroxidase or alkaline phosphatase, washing excess protein complex that is not bound to biotin from the system, and then adding to the system a substrate for the enzyme of the protein complex, whereupon the enzyme present in the system catalyzes a chromogenic reaction, wherein a colored product, which can be detected visually or spectrophotometrically, is produced. A similar system, wherein the label covalently joined to the nucleic acid is a "hapten" that is an inhibitor of the enzyme carbonic anhydrase and the signal-generating protein complex is an homopolymer of carbonic anhydrase or an heteropolymer of carbonic anhydrase with another protein (such as an enzyme) is described in European Patent Application Publication No. 0 210 021.

These indirect detection methods disadvantageously require a step of binding a signal-generating protein complex to nucleic acid probe. This step not only adds to the difficulty of carrying out an hybridization assay, by adding manipulations required to carry out the assay, but also reduces the sensitivity of the assay because of the unavoidable increase in background due to non-specific binding of a protein complex in an assay system. For example, when protein complexes comprising avidin or streptavidin are employed with biotin-derivatized nucleic acids as probes in hybridisation assays, sensitivity is significantly limited by the high level of background which results from the binding of avidin or streptavidin to not only biotinylated nucleic acid probes but also endogenous biotinylated proteins and various glycoproteins which are commonly found in clinical samples.

Heretofore, direct, nonradioactive, enzymatic systems for the detection of nucleic acid probes, i.e. systems wherein the probe is a nucleic acid covalently joined to a catalytically active, signal-generating enzyme, usually prior to hybridization of the nucleic acid with target, have rarely been employed.

Renz and Kurz, Nucl. Acids Res. 12, 3435 - 3444(1984), have described DNA probes wherein single-stranded DNA (i.e., heat-denatured plasmid DNA) is coupled covalently with glutaraldehyde to a complex of calf intestine alkaline phosphatase or horseradish peroxidase. The enzyme complex is prepared by attaching molecules of the enzyme to a polyethyleneimine core. The enzyme in the probe remains capable of catalyzing a chromogenic reaction, whereby the probe is detected. The probes described by Renz and Kurz, supra, require a single-stranded nucleic acid of at least about 1 kilobase in length and have limited sensitivity because of the high level of background found when they are used in hybridization assays.

Jablonski et al., Nucl. Acids Res. 14, 6115 - 6128 (1986), describe probes made by conjugating calf intestine alkaline phosphatase to synthetic oligonucleotides (21 - 26 bases in length), wherein one of the bases is uracil modified to have covalently joined to carbon-5 a 12-atom moiety with a free amino group. The enzyme is covalently linked to an oligonucleotide by first reacting oligonucleotide with disuccinimidyl suberate, whereby one of the carboxy groups of the suberate is joined in an amide linkage to the free amino group joined to the modified uracil of the oligonucleotide, and then reacting the modified oligonucleotide with alkaline phosphatase, whereby the other carboxy group of the suberate forms an amide with a free amino group of the enzyme. Jablonski et al., supra, report complications in the synthesis of the probes, apparently due to the instability of the N-hydroxy succinimidyl groups of the disuccinimidyl suberate linker.

Li et al., Nucl. Acids Res. 15, 5275 - 5287 (1987) report preparation and use of nucleic acid probes wherein calf intestine alkaline phosphatase is linked covalently to a single-stranded oligonucleotide through a sulfhydryl. In the probes reported by Li et al., supra, single-stranded oligonucleotide is derivatized at the N⁴-position of a cytidine at the 3'-end of a spacer of several nucleotides, which are added to the 3'-end of the oligonucleotide segment, whose sequence is complementary to that of the target segment and to which the probe is intended to hybridize in an hybridization assay. The group derivatized to the 3'-cytidine has a free sulhydryl group. In the conjugation reaction linking the oligonucleotide to the enzyme, the free sulhydryl joined to the oligonuculeotide reacts with a bromoacetyl group that had been joined to the alkaline phosphatase in a reaction between the enzyme and the N-hydroxysuccinimide ester of alpha-bromoacetic acid. The probes and their method of preparation reported by Li et al., supra, suffer from the disadvantageous sensitivity of sulhydryl-containing compounds to oxidizing conditions, whereby unwanted disulfides form. See, e.g., Orgel and Chu, Nucl. Acids Res. 16, 3671 - 3691 (1988). Further, the multi-nucleotide spacer in the probes of Li et al. can adversely affect the specificity of the probes.

King et al., Biochemistry 25, 5774 - 5779 (1986), describe a method for preparing conjugates of one protein with another protein. According to King et al., after ovalbumin is modified to have either aryl aldehyde or acyl hydrazide groups, the aldehyde- and hydrazide-group-modified ovalbumins are coupled to form oligomeric conjugates wherein the ovalbumins are covalently joined by linkers that comprise an hydrazone group. Reduction of the hydrazone group of the linkers to an hydrazide group using cyanoborohydride is also described.

Kremsky et al., Nucl. Acids Res. 15, 2891 - 2909 (1987), describe preparation of a single-stranded oligonucleotide (of 16 bases) derivatized at the 5'-carbon of the 5'-nucleotide with a moiety of formula -(PO₄)(CH₂)₁₁(CO)(NH)(CHO), with a free aldehyde group. Kremsky et al., supra, describe the reaction of this oligonucleotide with biotin hydrazide (i.e., biotin with the carboxylate group modified to a group of formula -(CO)(NH)(NH₂)) and with polystyrene latex microspheres derivatized with groups of formula -(CO)(NH)(NH₂), whereby the oligonucleotide becomes covalently linked to biotin and the latex microspheres, respectively, by a linker which comprises an hydrazide group, formed through cyanoborohydride reduction of an intermediate wherein the linker comprises an hydrazone in place of the hydrazide. Kremsky et al., supra, suggest that the linker with an hydrazone group cannot be made in significant yield in a reaction between an aldehyde group and an hydrazino group and that successful conjugation between an aldehyde and an hydrazino requires the presence of cyanoborohydride to drive reduction of hydrazone to hydrazide. Kremsky et al., supra, indicate that the single-stranded oligonucleotide in the biotin-derivatized and latex microsphere-derivatized conjugates, with linker comprising an hyrazide group, hybridizes to a nucleic acid segment of complementary sequence.

The present invention entails a novel nucleic acid probe, which comprises a single-stranded nucleic acid covalently joined, from the 5'-carbon of the 5'-terminal nucleotide, to a catalytically active enzyme by a linker that comprises an hydrazone group, of formula -NH-N=CH-, or an hydrazide group, of formula -NH-NH(CH₂)-. The probes according to the invention hybridize with unexpected speed, efficiency and specificity to target nucleic acids. Further, the sensitivity of the probes according to the invention is surprisingly good, especially for the probes wherein the nucleic acid is an oligonucleotide of fewer than about 150 bases in length in the sequence complementary to that of the target segment.

Consequently, the invention also entails surprisingly advantageous, improved nucleic acid probe hybridization assays, wherein a direct detection system is employed.

Further, in another aspect, the invention entails a novel, single-stranded nucleic acid, which is an intermediate for preparing a nucleic acid probe according to the invention and which is derivatized, at the 5'-carbon of the 5'-terminal nucleotide, with a moiety with a free hydrazino group, of formula -NH(NH₂).

Still further, the invention entails a surprisingly high-yield and surprisingly efficient method for making a probe according to the invention which comprises reacting a nucleic acid according to the invention with a catalytically active enzyme that is derivatized to have a free aldehyde group, of formula -(CO)H.

The probes according to the invention, wherein the linker between the terminal nucleotide of the nucleic acid and the enzyme comprises an hydrazone group, are surprisingly stable. In addition, however, the invention entails a method for making a probe according to the invention, wherein the linker between the terminal nucleotide of the nucleic acid and the enzyme comprises an even more stable hydrazide group, which method comprises reducing with cyanoborohydride a probe according to the invention, wherein the linker comprises an hydrazone group. This method is surprisingly advantageous in its unexpected specificity, whereby the nucleic acid and the enzyme are not substantially affected and the only group that is substantially affected is the hydrazone group in the linker.

Still further the invention entails the compound 4-N'-benzylamidobenzaldehyde and nucleic acid probes derivatized with that compound and with 2,4-dinitrobenzaldehyde.

In one aspect, the present invention entails a nucleic acid probe which comprises a single-stranded nucleic acid and a catalytically active enzyme, wherein said nucleic acid comprises a segment of at least 20 nucleotides in the sequence complementary to the sequence of the target segment of said probe; wherein said nucleic acid and said enzyme are linked by a linker of a formula selected from the group consisting of formula I

-(PO₃)NH(R₁)N=CH(R₂)(CO)- I

and formula II

-(PO₃)NH(R₁)NH(CH₂)(R₂)(CO)- II

wherein R₁ is selected from the group consisting of a bond between the nitrogens, (NH)(CO)(NH), 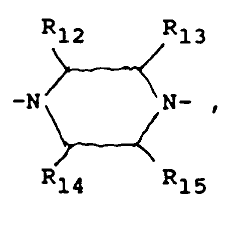
and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein R₂ is selected from the group consisting of alkylene of 1 to 20 carbon atoms and arylene of formula: 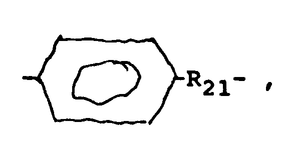
wherein R₂₁ is alkylene of 0 to 10 carbon atoms; wherein the -(PO₃)- group is bonded to the 5'-carbon of the 5'-nucleotide of the nucleic acid; and wherein the carbons of the carbonyl groups at the right-hand sides of figures I and II are bonded to the nitrogen of a free amino group of the enzyme.

In a further aspect, the invention entails a nucleic acid probe which comprises a single-stranded nucleic acid and a catalytically active enzyme, wherein said nucleic acid comprises a segment of at least 20 nucleotides in the sequence complementary to the sequence of the target segment of said probe; wherein said enzyme is a glycoprotein that has been oxidized by treatment with periodate; wherein said nucleic acid and said enzyme are linked by a linker of a formula selected from the group consisting of formula IX

-(PO₃)NH(R₁)N= IX

and formula X

-(PO₃)NH(R₁)NH- X

wherein R₁ is selected from the group consisting of a bond between the nitrogens, (NH)(CO)(NH), 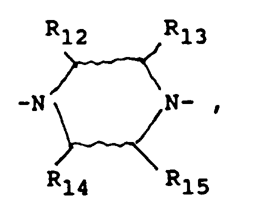
and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein the -(PO₃)- group is bonded to the 5'-carbon of the 5'-nucleotide of the nucleic acid; and wherein the -N= at the right-hand side of formula IX and the nitrogen of the -NH- group at the right-hand side of formula X are bonded to a carbon, of a sugar residue of the enzyme, that was the carbon of a carbonyl group formed in the treatment of the enzyme with periodate.

In still another aspect, the present invention encompasses a single-stranded nucleic acid which is an intermediate for making a nucleic acid probe for detecting a target segment, comprises a segment of at least 20 nucleotides in the sequence complementary to that of said target segment, and is derivatized with a moiety of a formula XIV

-(PO₃)(NH)(R₁)NH₂ XIV

wherein R₁ is selected from the group consisting of a bond between the nitrogens, (NH)(CO)(NH), 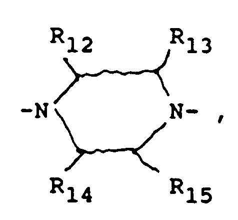
and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; and wherein the -(PO₃)- group is bonded to the 5'-carbon of the 5'-nucleotide of the nucleic acid.

In a still further aspect, the invention entails a method of making a nucleic acid probe which comprises a single-stranded nucleic acid and a catalytically active enzyme, wherein said nucleic acid comprises a segment of at least 20 nucleotides in the sequence complementary to the sequence of the target segment of said probe; wherein said nucleic acid and said enzyme are linked by a linker of formula I:

-(PO₃)NH(R₁)N=CH(R₂)(CO)- I

wherein R₁ is selected from the group consisting of a bond between the nitrogens, (NH)(CO)(NH), 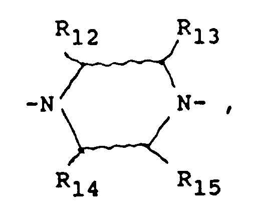
and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein R₂ is selected from the group consisting of alkylene of 1 to 20 carbon atoms or arylene of formula: 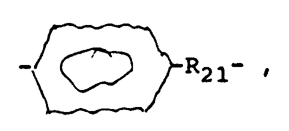
wherein R₂₁ is alkylene of 0 to 10 carbon atoms; wherein the -(PO₃)- group is bonded to the 5'-carbon of the 5'-nucleotide of the nucleic acid; and wherein the carbon of the carbonyl group at the right-hand side of figure I is bonded to the nitrogen of a free amino group of the enzyme, which method comprises reacting, in an aqueous solution buffered at a pH between about 5 and about 9 (preferably 7.0 to 9.0), a single-stranded nucleic acid, which has the same sequence as that of the nucleic acid probe and is derivatized with a moiety of a formula XIV

-(PO₃)(NH)(R₁)NH₂ XIV

wherein the -(PO₃)- group is bonded to the 5'-carbon of the 5'-nucleotide, with a catalytically active enzyme, which is derivatized at a free amino group with a moiety of formula XVIII

(CHO)(R₂)(CO)- XVIII.

In another aspect, the invention further entails a method of making a nucleic acid probe which comprises a single-stranded nucleic acid and a catalytically active enzyme, wherein said nucleic acid comprises a segment of at least 20 nucleotides in the sequence complementary to the sequence of the target segment of said probe; wherein said enzyme is a glycoprotein that has been oxidized by treatment with periodate; wherein said nucleic acid and said enzyme are linked by a linker of a formula IX

-(PO₃)NH(R₁)N= IX

wherein R₁ is selected from the group consisting of a bond between NH and N, (NH)(CO)(NH), 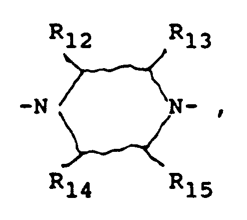
and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein the -(PO₃)- group is bonded to the 5'-carbon of the 5'-nucleotide of the nucleic acid; and wherein the -N= at the right-hand side of formula IX is bonded to a carbon, of a sugar residue of the enzyme, that was the carbon of a carbonyl group formed in the treatment of the enzyme with periodate, which method comprises reacting, in an aqueous solution buffered at a pH between about 5 and about 9 (preferably 6.0 to 8.0), a single-stranded nucleic acid, which has the same sequence as that of the nucleic acid probe and is derivatized with a moiety of a formula XIV

-(PO₃)(NH)(R₁)NH₂ XIV

wherein the -(PO₃)- group is bonded to the 5'-carbon of the 5'-nucleotide, with said catalytically active enzyme.

In still another aspect, the invention encompasses a method of making a first nucleic acid probe which comprises a single-stranded nucleic acid and a catalytically active enzyme, wherein said nucleic acid comprises a segment of at least 20 nucleotides in the sequence complementary to the sequence of the target segment of said probe; wherein said nucleic acid and said enzyme are linked by a first linker of formula II:

-(PO₃)NH(R₁)NH(CH₂)(R₂)(CO)- II

or formula XXIX

-(PO₃)NH(R₁)NH- XXIX

wherein R₁ is selected from the group consisting of a bond between the nitrogens, (NH)(CO)(NH), 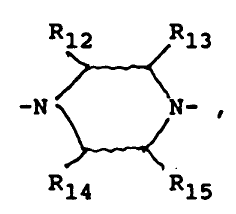
and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein R₂ is selected from the group consisting of alkylene of 1 to 20 carbon atoms and arylene of formula: 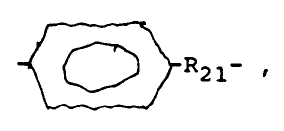
wherein R₂₁ is alkylene of 0 to 10 carbon atoms; and wherein the -(PO₃)- group is bonded to the 5'-carbon of the 5'-nucleotide of the nucleic acid; provided that, if the formula of said first linker is formula II, the carbon of the -(CO)- group at the right-hand side of said formula is bonded to a free amino group of the enzyme; and provided further that, if the formula of said first linker is formula XXIX, the catalytically active enzyme is a glycoprotein and has been oxidized with periodate and the nitrogen of the -NH- group at the right-hand side of said formula is bonded to a carbon, of a sugar residue of the enzyme, that was the carbon of a carbonyl group formed in the treatment of the enzyme with periodate; which method comprises reacting, in an aqueous solution buffered at a pH of about 5 to about 9 (preferably 5.0 to 6.0), an alkali metal salt of cyanoborohydride with a second nucleic acid probe which is substantially the same as said first nucleic acid probe except that, in said second probe, the nucleic acid and enzyme are linked by a second linker of formula I

-(PO₃)NH(R₁)N=CH(R₂)(CO)- I

or formula IX

-(PO₃)NH(R₁)N= IX

in place of said first linker, provided that, if said first linker is of formula II, said second linker is of formula I, and, if said first linker is of formula XXIX, said second linker is of formula IX.

In yet a further aspect, the invention entails an improved nucleic acid probe hybridization assay for a nucleic acid analyte, which comprises a target segment of at least 20 nucleotides in a known sequence, wherein the improvement comprises employing as the nucleic acid probe for detecting nucleic acid analyte in said assay a probe which comprises a single-stranded nucleic acid and a catalytically active enzyme, wherein the nucleic acid of the probe comprises a segment with the sequence complementary to the sequence of said target segment; wherein said nucleic acid and said enzyme are linked by a linker of a formula selected from the group consisting of formula I

-(PO₃)NH(R₁)N=CH(R₂)(CO)- I

formula II

-(PO₃)NH(R₁)NH(CH₂)(R₂)(CO)- II

formula IX

-(PO₃)NH(R₁)N= IX

and formula X

-(PO₃)NH(R₁)NH- X

wherein R₁ is selected from the group consisting of a bond between the nitrogens, (NH)(CO)(NH), 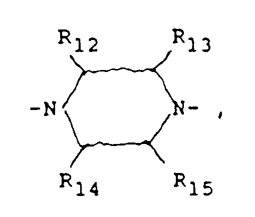
and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein R₂ is selected from the group consisting of alkylene of 1 to 20 carbon atoms and arylene of formula: 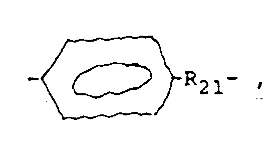
wherein R₂₁ is alkylene of 0 to 10 carbon atoms; wherein the -(PO₃)- group is bonded to the 5'-carbon of the 5'-nucleotide of the nucleic acid; and wherein the carbons of the carbonyl groups at the right-hand sides of figures I and II are bonded to the nitrogen of a free amino group of the enzyme; provided that, if the linker is of formula IX or formula X, the catalytically active enzyme is a glycoprotein that has been oxidized with periodate and -N= at the right-hand side of formula IX and the nitrogen of the -NH- group at the right-hand side of formula X are bonded to a carbon, of a sugar residue of the enzyme, that was the carbon of a carbonyl group formed in the treatment of the enzyme with periodate.

The invention also includes the use of a probe of the invention or nucleic acid of the invention in a hybridization assay or in making a probe, respectively.

Reference herein to a "probe of the invention," is to a probe of the invention that comprises an catalytically active enzyme.

As used herein, "nucleic acid" means a DNA or an RNA, unless the context in which the term is used limits its meaning to only one of the two. Similarly, "polynucleotide" and "oligonucleotide" mean a nucleic acid of at least 8 nucleotides in length, unless the context in which the terms are used limits their meanings to only one of a DNA and RNA. "Nucleotide" means a ribonucleotide or a 2'-deoxyribo-nucleotide, unless the context in which the term is used limits its meaning to only one of the two. Reference to "5'-nucleotide" of a single-stranded nucleic acid is to the nucleotide at the 5'-end of the nucleic acid.

The nucleic acids of the invention and the nucleic acid components of the nucleic acid probes of the invention are preferably DNAs. More preferably, they are DNAs that are oligonucleotides that can be provided readily in pure form in significant quantities by automated, stepwise, solid-phase synthetic methods, i.e., up to about 150 nucleotides in length.

A "nucleic acid analyte" means a nucleic acid (single-stranded or double-stranded) whose presence in a sample is assayed for in a nucleic acid probe hybridization assay. A nucleic acid analyte comprises a single-stranded "target segment," to which a nucleic acid probe, that is intended to be used to detect the analyte in a nucleic acid probe hybridization assay, hybridizes by complementary base-pairing. Thus, the nucleic acid component of a nucleic acid probe comprises a segment (referred to herein as the "hybridizing segment") with a sequence such that, under the stringency conditions employed in the hybridizations and washings of the hybridization assay in which the probe is to be employed, the hybridizing segment will hybridize, with a specificity deemed to be acceptable for the assay, to a target segment of the analyte of the assay (if such analyte happens to be present in a sample on which an assay is carried out). The sequence of the hybridizing segment of the nucleic acid component of a nucleic acid probe is determined by the sequence of the target segment of the nucleic acid analyte, which the probe is used to detect. To maximize specificity of a nucleic acid probe for its pre-selected analyte in hybridization assays, the sequence of the hybridizing segment of the nucleic acid component of the probe will be complementary to the sequence of the target segment. Also, to maximize such specificity, the target segment of analyte is preselected according to sequence to attempt to assure that hybridization of nucleic acid probe to target segment in an assay occurs detectably if and only if analyte is present in the sample being assayed. Thus, a target segment will always be at least about 8 nucleotides in length and more prefereably at least about 20 nucleotides and less than about 50 nucleotides in length. Further, preferably, in a nucleic acid or nucleic acid component of a probe according to the present invention, the "hybridizing segment," in addition to being complementary in sequence to that of the corresponding, pre-selected target segment, will be coincident with the whole nucleic acid, i.e., the sequence of the nucleic acid will consist of the sequence of the hybridizing segment which, in turn, will be complementary to the sequence of the corresponding, pre-selected target segment.

A "catalytically active enzyme" is an enzyme which, in a probe according to the invention, retains the ability to catalyze a reaction, which the enzyme catalyzes when not a part of such a probe. Preferably a catalytically active enzyme in a probe according to the invention retains at least about 50 % of the activity of the free, unmodified enzyme. For those of ordinary skill, it is straighforward to measure the activity of an enzyme in a probe according to the invention to ascertain whether it is catalytically active. Essentially, such a measurement is carried out on the probe in the same way that it is carried out on free, unmodified enzyme, for which, by definition, a catalyzed reaction and how to measure the catalytic activity of the enzyme in such reaction will be known. For an enzyme in a probe according to the invention to retain its catalytic activity, it is necessary that the enzyme retain its catalytic activity, after modification to have, or be covalently joined to, one or more free aldehyde groups but before being covalently joined to the nucleic acid component of the probe.

Preferred among the enzymes which can be employed as part of a probe according to the invention are enzymes which catalyze reactions that yield products that are easily detectable, most preferably spectrophotometrically (e.g., by measurement of fluorescence emission or light absorbtion) or by simple visual observation. Thus, enzymes are preferred that catalyze chromogenic reactions, which yield, from substrates that have little or no absorbtivity in the visible range of wavelengths of light, products that are intensely colored, by virtue of intense flourescence emission or high absorbtivity at a wavelength in the visible range of wavelengths. Many such enzymes are known that are suitable for use as the enzyme components in probes according to the invention. These include phosphatases, such as calf intestine alkaline phosphatase, E. coli alkaline phosphatase, and potatoe acid phosphatase; peroxidases, such as horseradish peroxidase and bovine milk lactoperoxidase; luciferases, such as those from Photobacterium fischeri or firefly; ureases, such as that from jack bean; carbonic anhydrases, such as that from mammalian (e.g., bovine) erythrocytes; beta-galactosidases, such as those from E. coli and Aspergillus oryzae; and oxidases, such as glucose oxidase from Aspergillus niger and alcohol oxidase from Pichia pastoris. Most preferred among the enzymes suitable for use as the enzyme component in a probe according to the invention are calf intestine alkaline phosphatase and horseradish peroxidase; among these two, the alkaline phosphatase is the more preferred.

Reference herein to a "free amino group" of an enzyme or other protein means an unmodified epsilon amino group of a lysine of the enzyme or other protein or the unmodified amino-terminal amino group of the enzyme or other protein (or a subunit of the enzyme or protein, if it has more than one subunit).

Reference herein to a "linker comprising an hydrazone group" or a "linker moiety comprising an hydrazone group" means a moiety which links the 5'-carbon of the 5'-terminal nucleotide of a nucleic acid to an enzyme molecule, a 2,4-dinitrophenyl group or a 4-N-benzylamidophenyl group and which includes a group of formula -(NH)N=CH-, wherein the carbon is (A) covalently joined to the enzyme by a group of formula XX

-(R₂)(CO)- XX

wherein R₂ is selected from the group consisting of alkylene of 1 to 20 carbon atoms and arylene of formula: 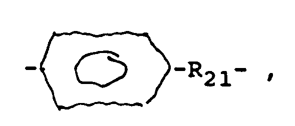
wherein R₂₁ is alkylene of 0 to 10 carbon atoms and wherein the carbonyl group is bonded, as part of an amide group, to a nitrogen that is the nitrogen of a group that was a free amino group of the enzyme, (B) is a carbon of a sugar residue of the enzyme, that was the carbon of a carbonyl group formed in the treatment of the enzyme with periodate, or (C) is covalently joined to a 2,4-dinitrophenyl group or 4-N'-benzylamidophenyl group.

Reference herein to a "linker comprising an hydrazide group" or a "linker moiety comprising an hydrazide group" means a moiety which links the 5'-carbon of the 5'-terminal nucleotide of a nucleic acid to an enzyme molecule, a 2,4-dinitrophenyl group or a 4-N'-benzylamidophenyl group and which includes a group of formula -(NH)(NH)(CH₂)-, wherein the carbon is (A) covalently joined to the enzyme by a group of formula XX

-(R₂)(CO)- XX

wherein R₂ is selected from the group consisting of alkylene of 1 to 20 carbon atoms and arylene of formula: 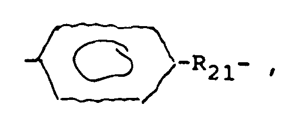
wherein R₂₁ is alkylene of 0 to 10 carbon atoms and wherein the carbonyl group is bonded, as part of an amide group, to a nitrogen that is the nitrogen of a group that was a free amino group of the enzyme, (B) is a carbon of a sugar residue of the enzyme, that was the carbon of a carbonyl group formed in the treatment of the enzyme with periodate, or (C) is covalently joined to a 2,4-dinitrophenyl group or 4-N'-benzylamidophenyl group. A probe of the invention (including an indirect probe) with a "linker comprising an hydrazide group" joining the nucleic acid and enzyme (or 2,4-dinitrophenyl or 4-N-benzylamidophenyl) is made by reduction with cyanoborohydride of a probe according to the invention which is the same but for having an hydrazone group in place of the hydrazide group in the linker.

A nucleic acid of the invention, which is single-stranded, an intermediate for making a probe (including an indirect probe) according to the invention and derivatized at the 5'-carbon of the 5'-terminal nucleotide with a moiety of formula XIV

-(PO₃)(NH)(R₁)NH₂ XIV

wherein R₁ is selected from the group consisting of a bond between the nitrogens, (NH)(CO)(NH), 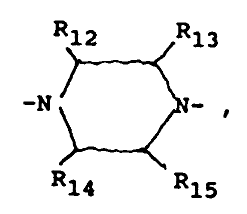
and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; and wherein the -(PO₃)- group is bonded to the 5'-carbon of the 5'-nucleotide of the nucleic acid, is prepared as follows: First, the sequence of the nucleic acid is determined with reference to the target segment to which the probe, which is to be made with the nucleic acid, is to hybridize in nucleic acid probe hybridization assays; the nucleic acid will comprise a segment with the sequence complementary to that of said target segment. Nucleic acids according to the invention preferably consist of a segment complementary in sequence to that of the corresponding target segment (i.e., have the same number of bases as the target segment in the sequence complementary to that of the target segment) and have 20 - 50 bases.

Once the sequence of a nucleic acid according to the invention has been determined, an unmodified nucleic acid with the sequence is prepared by any of numerous methods known in the art. Thus, for example, a bacteriophage, such as M13 or Q-beta, which has a single-stranded genome, can be prepared to have a genome which comprises a segment with the sequence complementary to the pre-selected target segment sequence, the genome of such phage can be isolated and, if closed circular, linearized by sonication which fragments the genome into fragments with an average size larger than that of the segment with the sequence complementary to that of the target segment. RNA which comprises a segment of defined sequence can be provided in vitro by run-off transcription from a promoter of, e.g., a bacteriophage DNA-dependent RNA polymerase such as the T7, SP6 or T3 RNA polymerase, of a DNA with a segment which has the sequence complementary to that desired for the RNA. More preferably, however, the unmodified nucleic acid of desired sequence will be synthesized by any of numerous solid-phase methods known in the art, such as the well known cyanoethyl phosphoramidite method, coupled with any standard, chromatographic technique to isolate the desired nucleic acid from the mixture that results from such syntheses; preferably the synthesis will be carried using an automated nucleic acid synthesizer.

Once the desired, unmodified nucleic acid has been obtained, it is treated to be phosphorylated with monophosphate at the 5'-terminus, unless it had been so phosphorylated in the course of being prepared. With nucleic acid obtained from a solid phase synthetic technique, which has an hydroxyl group bonded to the 5'-carbon of the 5'-terminal nucleotide, the 5'-phosphorylation can be accomplished by kinasing with ATP and T4 polynucleotide kinase, as well known in the art. See Maniatis et al. Molecular Cloning: a Laboratory Manual, Cold Spring Harbor Laboratory Press, Cold Spring Harbor, New York (1982). After the kinasing, oligonucleotide is separated from unreacted ATP, pyrophosphate and enzyme by any standard technique. A preferred technique, referred to herein as "LiCl/ethanol precipitation," is carried out as follows: To 1 volume of the aqueous reaction mixture are added 3 volumes of absolute ethanol and 0.1 volume of 8 M LiCl to bring the LiCl concentration to about 0.2 M. The solution with LiCl is then cooled to -70 °C to precipitate the nucleic acid. The solution is then centrifuged to pellet the nucleic acid and the supernatant is removed and discarded.

The resulting nucleic acid, including the 5'-phosphorylated, is then treated by a method adapted from that described by Chu et al., Nucl. Acids Res. 11, 6513 - 6529 (1983), employing a dihydrazide, of formula

H₂N(R₁)NH₂ XV

wherein R₁ is selected from the group consisting of a bond between the nitrogens, (NH)(CO)(NH), 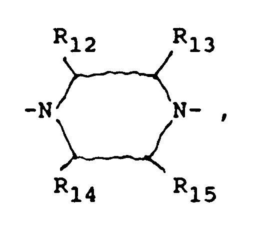
and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms, in place of the dialkylamine employed in the method described by Chu et al. (1983), supra, to make the nucleic acid according to the invention.

Briefly, the 5'-phosphorylated nucleic acid is converted to the 5'-phosphorimidazolide nucleic acid by treatment of the nucleic acid from the kinasing reaction with aqueous imidazole or methylimidazole (at about 0.05 M to 0.25 M) in the presence of a water soluble carbodiimide coupling agent, such as 1-ethyl-3-(3-dimethylaminopropyl)-carbodiimide ("EDC") or N-cyclohexyl-N'-2-(4'-methyl-morpholinium)ethyl carbodiimide p-toluene sulfonate (also at about 0.05 M to about 0.25 M), at pH 5.5 - 6.8 and room temperature for 30 minutes to 2 hours. Preferred reaction conditions are about 0.1 M imidazole, about 0.15 M carbodiimide, pH 6.0, for 90 minutes. Oligonucleotide is then separated from other reagents and products by a standard technique for oligonucleotide isolation, of which one LiCl/ethanol precipitation is prefered.

To make the nucleic acid according to the invention, the mixture of nucleic acids, including the 5'-phosporimidazolide, is taken up in an aqueous solution of a dihydrazide of formula XV, at between about 0.05 M and about 0.5 M and at a pH between about 7 and about 10, and reaction is allowed to proceed at between about 30 °C and about 65 °C for between about 1 hour to about 24 hours. Preferably the reaction is carried out with about 0.25 M of the dihydrazide at a pH of about 8.5 and a temperature of about 50 °C for about 3 hours. Nucleic acid, including the nucleic acid according to the invention, is then isolated from excess dihydrazide reagent by a three consecutive LiCl/ethanol precipitations.

The preferred nucleic acids of the invention are made with hydrazine (wherein R₁ in formula XV is a bond between the nitrogens), carbohydrazide (wherein R₁ in formula XV is (NH)(CO)(NH), and adipic dihydrazide (wherein R₁ in formula XV' is (NH)(CO)(CH₂)₄(CO)(NH). The most preferred are made with carbohydrazide.

The nucleic acids of the invention are useful as intermediates for making probes according to the invention as described further below.

It is noteworthy that, after the kinasing reaction, the reaction to make the 5'-phosphorimidazolide derivative, and the reaction to make the nucleic acid of the invention (derivatized to have a free hydrazino group), there is, advantageously, no need to separate unreacted oligonucleotide (or derivative) from the derivative made in the reaction. This is because, unexpectedly, the dihydrazide of formula XV reacts detectably only at the phosphorus of the phosphorimidazolide derivative and, in the reaction, described below, of the nucleic acid of the invention with aldehyde-group-derivatized enzyme, reaction of the aldehyde group joined to the enzyme occurs detectably only at the nitrogen of the -NH₂ group of the hydrazino group of the nucleic acid of the invention. Further, a probe of the invention, because of the difference in its size from the sizes of the various oligonucleotides and derivatives thereof, is easily separated from unreacted oligonucleotides and derivatives by simple size-exclusion (e.g., gel permeation) chromatography. Thus, to assure that probe of the invention is isolated from oligonucleotide and oligonucleotide-derivative contaminants, advantageously all that is required is a size-based separation step after the reaction of aldehyde-group-derivatrized enzyme with hydrazino-group-derivatized nucleic acid of the invention.

With indirect probes of the invention, a simple HPLC step will separate probe from the other nucleic acid derivatives and low molecular weight compounds that occur during the synthesis and are not removed in LiCl/ethanol precipitation steps.

Aldehyde-group-derivatized enzyme, for use in making a probe according to the invention, can be made following King et al., supra. Thus, the enzyme is dissolved in an aqueous solution, buffered at a pH between about 7.5 and about 9.0 (preferably about 8.5), to a concentration of typically about 10 µM to about 100 µM. To the enzyme solution is added, usually in a dry organic solvent such as acetonitrile and at a concentration in the organic solvent between about 100 and about 1000 times greater than that of the enzyme in the enzyme solution, a 1-fold to 100-fold (preferably 7.5-fold to 70-fold, most preferably about 50-70 fold) molar excess (relative to the amount of enzyme in the enzyme solution) of an activated ester (e.g., N-hydroxysuccinimide ester) of a compound of formula XVI

H(CO)(R₂)(CO₂H) XVI,

wherein R₂ is selected from the group consisting of alkylene of 1 to 20 carbon atoms and arylene of formula: 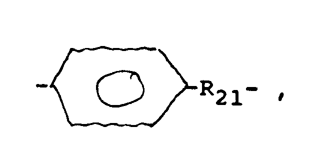
wherein R₂₁ is alkylene of 0 to 10 carbon atoms. ("Alkylene of 0 carbon atoms" means that R₂₁ is simply part of the bond from the phenyl group to the carboxylate group of the compound of formula XVI). The reaction of the activated ester with free amino group(s) of the enzyme is allowed to continue for about 10 minutes to about an hour at a temperature between about 4 °C and about 30 °C. Finally, excess reagent and low molecular weight products of the reaction are removed from the enzyme by dialysis, at about 0 °C to about 10 °C, of the reaction solution against an aqueous buffer, preferably one, at a pH between about pH 7 and pH 9, that is suitable for the conjugation reaction in which probe is made by reaction of hydrazino-group-derivatized oligonucleotide with the aldehyde-group-derivatized enzyme. In this regard, preferred buffering agents will be non-nucleophilic; among these are sulfonated buffering agents well known in the art, such as MOPS, PIPES (piperazine -N,N'-bis(2-ethane sulfonic acid)), HEPES (N-2-hydroxyethylpiperazine-N'-2-ethanesulfonic acid), and HEPPS (N-2-hydroxyethylpiperazine-N-2-propanesulfonic acid) and the like. Preferably, prior to the dialysis, the reaction continues for about 30 minutes at room temperature. N-hydroxysuccinimido esters are preferred. In the most preferred activated ester, R₂ is p-phenylene (i.e., R₂₁ in formula XVI is 0 carbon atoms).

If the enzyme is a glycoprotein, it can be modified to have free aldehyde groups by an alternative procedure involving oxidation of sugar moieties of the glycoprotein. In particular, such an enzyme can be oxidized in aqueous solution with periodate whereupon aldehyde groups are formed at vicinal hydroxylated carbons of the sugar moieties, via the Malaprade reaction. Thus, employing an alkali metal salt of IO₄⁻ (preferably the Na⁺ salt), an aqueous solution of the enzyme, at a concentration between about 1 µM and about 100 µM (preferably about 50 µM) and a pH of between about 5 and about 8 (preferably in distilled water and at a pH of about 6 to about 7), is combined with an aqueous solution of the IO₄⁻ salt to provide an initial concentration of IO₄⁻ in the combined solutions of between about 10 mM and 100 mM (preferably about 500 times to about 1000 times the concentration of the enzyme). The oxidation reaction is allowed to proceed for about 10 minutes to about an hour at between about 4 °C and 30 °C (preferably about 20 minutes at room termperature) and, finally, excess periodate and low molecular weight products of the oxidation reaction are removed from the enzyme by dialysis against an aqueous buffer at a pH of between about 4 and about 8 and a temperature of between about 0 °C and about 30 °C. In a preferred procedure, a first dialysis is carried out at about 4 °C against a buffer at a pH of about 4 to about 5 (e.g., sodium acetate buffer, pH 4.5) and then a second dialysis is carried out, also at about 4 °C, to change the enzyme solution to that in which the conjugation reaction with hydrazino-group-derivatized nucleic acid is to be carried out with the enzyme (i.e., a solution buffered to a pH of about 7 to about 9 with a preferably non-nucleophilic, sulfonated buffering agent such as MOPS, PIPES, HEPES or HEPPS).

Many enzymes made by eukaryotic organisms are glycosylated and can be oxidized as described above with periodate to have free aldehyde groups while maintaining their catalytic activity. Especially noteworthy among these enzymes are eukaryotic peroxidases, such as horseradish peroxidase and mammalian milk lactoperoxidases.

For a glycosylated enzyme, which can be derivatized to have free aldehyde groups either by reaction with an activated ester of a compond with an aldehyde or by oxidation with periodate, it appears that catalytic activity is reduced significantly less when the derivatization is with an activated ester than when it is with periodate oxidation. On the other hand, it appears that the activated ester-derivatized enzyme couples somewhat less efficiently than the periodate-oxidized enzyme with hydrazino-group-derivatized nucleic acid in the conjugation reaction to make a probe according to the invention.

Because of the surprising specificity of the free hydrazino group of a nucleic acid according to the invention for the free aldehyde group(s) on an enzyme derivatized with an activated ester of a compound with an aldehyde group or with periodate oxidation and the surprising specificity of such free aldehyde group(s) on the enzyme for the free hydrazino group of the nucleic acid, it is advantageously not necessary, prior to conjugation reaction with hydrazino-group-derivatized nucleic acid, to separate enzyme that is modified to have free aldehyde group(s) from enzyme that is not so modified in the reaction with the activated ester or periodate.

A probe according to the invention, wherein the linker moiety, which joins the 5'-carbon of the 5'-nucleotide of the nucleic acid with the enzyme, comprises an hydrazone group is made by simply reacting, for about 0.5 hours to about 48 hours at a temperature between about 4 °C and about 35 °C (preferably about 16 hours at room temperature), in an aqueous solution buffered (preferably with a non-nucleophilic buffering agent such as a sulfonated buffering agent such as MOPS, PIPES, HEPES or HEPPS or the like) to a pH between about 5 to about 9 (preferably about 7 to about 8), the nucleic acid according to the invention, wherein the nucleic acid is the nucleic acid component of the probe, with the enzyme, which is the enzyme component of the probe and has been derivatized to have a free aldehyde group. Free aldehyde-group-derivatized enzyme reactant will be present initially in the solution at between about 10 µM to about 100 µM concentration (preferably about 25 µM to about 75 µM) and at a molar excess, relative to hydrazino-group-derivatized nucleic acid of the invention, of about 1 x (i.e., equimolar) to about 10 x.

The conjugation reaction provides probe with hydrazone group-containing linker between nucleic acid and enzyme or, in the case of indirect probe, 2,4-dinitrophenyl and 4-N-benzylamidophenyl, in surprisingly high yield, particularly in view of the teaching of Kremsky et al., supra, that stable hydrazone-containing linkers would not form in the reaction and that cyanoborohydride reducing agent would need to be present in the conjugation reaction mixture to achieve significant coupling between nucleic acid and enzyme or 2,4-dinitrophenyl or 4-N'-benzylamidophenyl.

The conjugation reaction is terminated and probe according to the invention and unreacted enzyme are separated from unreacted nucleic acid by size exclusion chromatography (e.g., gel filtration chromatography) at about 0 °C to about 10 °C (preferably 4 °C) using a buffer at a pH of about 7.5 to about 9.5 (e.g., 0.05 M Tris, pH 8.5) as eluant. Many materials suitable as matrices for size exclusion chromatography of proteins and nucleic acids are known in the art and are suitable for separating probe and unreacted enzyme from unreacted nucleic acid. As the skilled will understand, the material employed will depend in part on the molecular weights of the enzyme and nucleic acid components of the probe according to the invention, because the material must separate probe and unreacted enzyme from unreacted nucleic acid primarily on the basis of molecular weight differences. With respect to gel filtration chromatography with probes wherein calf intestine alkaline phosphatase is the enzyme and an oligonucleotides of fewer than about 150 bases is the nucleic acid component, a suitable material for a matrix for gel filtration chromatography is BioRad P-100, available from BioRad Laboratories, Inc., Richmond, California, USA. Similarly, Sephadex G-75, from Pharmacia, Inc., Piscataway, New Jersey, USA, is a suitable material for gel filtration separation from unreacted oligonucleotide of horseradish peroxidase and probes wherein horseradish peroxidase is the enzyme and an oligonucleotide of fewer than about 100 bases is the nucleic acid component.

Following the size exclusion chromatography to separate probe and unreacted enzyme from unreacted oligonucleotide, probe is resolved cleanly from unreacted enzyme by a chromatographic step wherein separation is based on charge. At basic pH's (e.g., about 7.5 to about 9.5, preferably about 8 to about 9), a probe according to the invention will be significantly more negatively charged than the unreacted enzyme that is combined with probe after the size exclusion chromatography step. Thus, for example, ion exchange chromatography can be employed to separate probe from unreacted enzyme. Again, the skilled will be aware of many resins suitable for ion exchange chromatographic separation of probe from unreacted enzyme and, for a given resin, enzyme and nucleic acid combination, will be capable easily of determining washing conditions suitable for separating unreacted enzyme from probe. Chromatography over DEAE cellulose (e.g., DE-52 from Whatman, Inc., Clifton, New Jersey, USA) using buffer at pH 8.5 (e.g., 0.1 M Tris) and first a wash with a gradient of increasing NaCl concentration (0 to 0.2 M NaCl) in the Tris buffer followed by washes with step-wise increasing NaCl concentration (e.g., 0.2 M, 0.5 M) in the Tris buffer has been found to be especially effective in simply and cleanly separating probe from unreacted enzyme, when the enzyme is calf intestine alkaline phosphatase. Similarly, DE-52 resin with 0.1 M Tris, pH 8.5, and washes of the Tris buffer with 0 M NaCl, 0.1 M NaCl and 0.2 M NaCl eluted unreacted horseradish peroxidase while a subsequent wash with the buffer with 0.5 M NaCl eluted probe, wherein the enzyme component was horseradish peroxidase.

It has also been possible, using DEAE cellulose in ion exchange chromatography with 0.1 M Tris, pH 8.5, as buffer and washes at step-wise increasing concentrations of NaCl to separate species of probe according to the invention wherein the numbers of nucleic acids per enzyme molecule differ, with the ratio of nucleic acid to enzyme increasing with increasing concentration of NaCl in the buffer used to elute the probe from the DEAE cellulose. Thus, for example, as described in more detail in the examples that follow, a "low salt" probe, wherein the ratio of nucleic acid to enzyme is 1:1 and which eluted from the DEAE cellulose at pH 8.5, 0.1 M Tris, 0.2 M NaCl, has been resolved from an "high salt" probe, wherein the ratio of nucleic acid to enzyme is 2:1 and which eluted from the DEAE cellulose at pH 8.5, 0.1 M Tris, 0.5 M NaCl, with probes wherein the nucleic acid component has about 30 bases and wherein the enzyme component is calf intestine alkaline phosphatase. The ratio of 2:1 to 1:1 probe made in the conjugation reaction between enzyme and nucleic acid depends on the number of free aldehyde groups joined to the enzyme employed in that reaction (with more aldehyde groups per enzyme molecule favoring an higher fraction of 2:1 probe) and the ratio of the concentration of aldehyde-group-derivatized enzyme to hydrazino-group-derivatized nucleic acid employed in the reaction (with a higher ratio favoring a lower fraction of 2:1 probe). Apparently also the G-C content of the nucleic acid influences the ratio of 2:1 to 1:1 probe, as oligonucleotides with a G-C content above about 45 % tend to yield a greater proportion of probe as 2:1 probe than oligonucleotides with lower G-C content.

It appears that, at least in nucleic acid probe hybridization assays carried out on filters (e.g., with dot blots, in Southern hybridizations), the sensitivity of 2:1 probe is essentially the same as that of 1:1 probe.

Probe of the invention wherein the linker joining enzyme and nucleic acid comprises an hydrazone group is very stable and reduction of the hydrazone group to an hydrazide group is not necessary to assure sufficient stability. However, if desired, probe of the invention, including indirect probe, wherein the linker joining enzyme and nucleic acid comprises an hydrazide group can be prepared as follows, in a simple reaction that is surprising in its specificity for reduction of the hydrazone group of the linker without observable effect on catalytic activity of the enzyme, or, in the case of indirect probe, on the 2,4-dinitrophenyl or 4-N'-benzylamidophenyl, and without observable effect on specificity and sensitivity of the probe. A solution of probe, wherein the linker comprises an hydrazone group, is taken up at a concentration of between about 0.5 µM and about 10 µM (preferably about 2 µM) in an aqueous solution buffered to a pH of between about 5.5 and about 7 (preferably about 6) and then an aqueous solution of an alkali metal salt of cyanoborohydride (preferably the sodium salt) at 0.1 M to 0.5 M (preferably 0.3 M to 0.5 M) is added to bring the initial concentration of cyanoborohydride in the probe solution to between about 0.01 M and 0.1 M (preferably about 0.02 M). The reduction is allowed to proceed at 4 °C to 35 °C (preferably at room temperature) for 1 hour to 24 hours (preferably about 16 hours) and, finally, the reaction mixture is exhaustively dialyzed to remove excess cyanoborohydride and bring the pH back to about 7.5 to about 9.5, preferably about 8.5.

The synthesis of the compound 4-N'-benzylamido-benzaldehyde is described in the examples that follow, as is the preparation of indirect probes of the invention, wherein the nucleic acid is derivatized at the 5'-carbon of the 5'-nucleotide with a moiety of formula XXIII or XXIV.

As understood in the art, with an indirect probe of the invention, detection is indirect. Such a probe is detected, for example, through binding, to the benzylamidophenyl or 2,4-dinitrophenyl group hapten of the probe, an antibody prepared against the group, which antibody has been coupled by a standard technique to a catalytically active enzyme. Then generation of an observable signal is by a reaction catalyzed by the enzyme coupled to the antibody.

The probes of the invention, which comprise an enzymatically active enzyme, are surprisingly sensitive in nucleic acid probe hybridization assays.

A probe of the invention can be employed advantageously as the detection probe (i.e., the probe which provides the basis for an observable signal in an assay system if nucleic acid analyte is present in the system) in any of the various types of nucleic acid probe hybridization assays known in the art. The probes of the invention, including the indirect probes, are used as detection probes in nucleic acid probe hybridization assays employing standard techniques that are well known in the art, see, e.g., Meinkoth and Wahl, Anal. Biochem. 138, 267 - 284 (1984) and PCT International Publication No. WO88/01302, incorporated herein by reference. Among the nucleic acid probe hybridization assays in which probes of the invention can be employed are filter-based assays, wherein nucleic acid of a sample being assayed is applied in single-stranded form directly to a support material, such as a nitrocellulose filter or nylon filter, and then detection probe is hybridized to nucleic acid analyte affixed to the filter prior to washing, to eliminate probe that has not stably hybridized to target segment or otherwise (as source of "background") become affixed to the filter, and then generation of signal using detection probe (directly or, in the case of an indirect probe, indirectly) that remains on the filter. Among such filter-based assays are those wherein nucleic acid of a sample is first separated in some way prior to application to the support material, as in Southern assays and Northern assays that are well known in the art. A probe of the invention, including an indirect probe, can also be employed as the detection probe in a sandwich assay, wherein analyte is first captured on a solid support (e.g., a macroporous material such as Sephacryl S-500 (Trade Mark) (Pharmacia) or a polystyrene latex material) by hybridization to a first probe ("capture probe") that is affixed to the solid support and that has the sequence complementary to that of a first target segment of the analyte and then a second probe (detection probe), that has the sequence complementary to that of a second target segment of analyte that does not overlap the first target segment, is hybridized to captured analyte prior to washing, to eliminate from the support detection probe that is not stably hybridized to second target segment of the analyte or otherwise (as source of "background") affixed to the filter, and then generation of signal using detection probe (indirectly or, in the case of probes of the present invention, directly). Methods of measuring "background signal" in these nucleic acid probe hybridization assays are well known; typically this is the signal from carrying out an assay on a sample of nucleic acid that is known to not contain nucleic acid analyte simultaneously with the assay of a sample thought to possibly contain analyte. As understood in the art, the presence of nucleic acid analyte in a sample of nucleic acid is indicated by a signal from the sample of a magnitude that exceeds the magnitude of the signal from the control sample, known to not contain analyte.

The invention is now illustrated in the following examples.

### EXAMPLE 1

### PREPARATION AND CHARACTERIZATION OF 5'-HYDRAZINO-DERIVATIZED AND 5'-HYDRAZONE-CONTAINING-LINKER-DERIVATIZED SINGLE-STRANDED NUCLEIC ACIDS

This example illustrates preparation of a single-stranded nucleic acid derivatized to have an hydrazino group or a linker comprising an hydrazone group covalently linked to the 5'-terminus (i.e., the 5'-carbon of the 5'-terminal nucleotide).

Oligonucleotides were synthesized using the solid-phase cyanoethyl phosphoramidite method in an automated DNA synthesizer (Model 380A, Applied Biosystems, Inc., Foster City, California, USA).

Two oligonucleotides so prepared are Oligonucleotides 85-133 and 87-416, which have the following sequences, which are complementary to sequences of segments of the insert coding for an hepatitis B virus surface antigen in plasmid pTB061B. (Plasmid pTB061B is plasmid pBR322 modified to have, in the EcoRI site, an approximately 900 base pair insert encoding the surface antigen of an hepatitis B virus of adw serotype.)
Oligo 85-133: 5'-TGGCTCAGTTTACTAGTGCCATTTGTTCAG
Oligo 87-416: 5'-AACCAATAAGAAGATGAGGCATAGCAGCA
Oligonucleotides from the synthesizer were purified as follows: Purification of the tritylated oligonucleotides was carried out using C-8 reverse-phase, semi-preparative chromatography (10 x 250 mm column) using a gradient of 15 - 35 % acetonitrile in 0.1 M triethylammonium acetate, pH 6.00, over 40 minutes and a flow rate of 3 ml/min. Then detritylation was carried out using 80 % acetic acid in water for 30 minutes, after which solvent was removed. Finally, oligonucleotides were isolated by LiCl/ethanol precipitation. The purified oligonucleotides migrated as single bands on a 20 % polyacrylamide gel and eluted as single peaks by reverse-phase HPLC analysis.

Purified oligonucleotides were phosphorylated at their 5'-ends following a standard procedure with ATP and T4 polynucleotide kinase. See Maniatis et al. Molecular Cloning: a Laboratory Manual, Cold Spring Harbor Laboratory Press, Cold Spring Harbor, New York (1982).

The 5'-phosphorylated oligonucleotides were converted to 5'-hydrazino-derivatized oligonucleotides as follows, by a procedure adapted from one reported by Chu et al., Nucl. Acids Res. 11, 6513 - 6529 (1983), for preparing 5'-amino-derivatized oligonucleotides:
Reaction tubes were silanized with a fresh 5 % solution of dichlorodimethyl silane in chloroform to prevent adhesion of nucleic acid to the walls of the tubes.

A 5'-phosphorylated oligonucleotide was converted to its activated, 5'-phosphorimidazolide derivative as follows: 8 nanomoles of the 5'-phosphorylated oligonucleotide were reacted for 90 minutes at 23 °C in 3 ml of 0.1 M imidazole, 0.15 M 1-ethyl-3-(3-dimethylaminopropyl)-carbodiimide ("EDC"), pH 6.00. The nucleic acid was then isolated by LiCl/ethanol precipitation.

The resulting nucleic acid, including the 5'-phosphorimidazolide derivative, was then taken up and reacted for 3 hours at 50 °C in 2.5 ml of a 0.25 M aqueous solution at pH 8.5 of hydrazine, carbohydrazide (formula: (NH₂NH(CO)NH(NH₂)), or adipic dihyrazide (formula: (NH₂NH(CO)(CH₂)₄(CO)NHNH₂) to make the 5'-hydrazino derivatives with a moiety of formula -(PO₃)NHNH₂, -(PO₃)NH(NH)(CO)(NH)NH₂, or -(PO₃)NHNH(CO)(CH₂)₄(CO)NHNH₂, respectively, covalently joined to the 5'-carbon of the 5'-terminal nucleotide of the nucleic acid.

A 5'-hydrazino-derivatized nucleic acid was isolated from excess hydrazine, carbohydrazide or adipic dihydrazide by three successive LiCl/ethanol precipitations.

Yields, relative to the 5'-phosphorylated oligonucleotide starting materials, were 60 - 75 %. Highest yields were obtained with the carbohydrazide.

The 5'-hydrazino-derivatized oligonucleotides were converted as follows to derivatives, wherein a 2,4-dinitro-phenyl ("DNP") group or 4-N'-benzylamidophenyl ("BAP") group was covalently linked to the 5'-carbon of the 5'-nucleotide by a linker of formula -(PO₃)NH(N=CH)-, comprising an hydrazone group: To 20 picomoles of the 5'-hydrazino-derivatized oligonucleotide in 5 µl of 50 mM MOPS (3-(N-morpholino)-propane sulfonic acid) buffer, pH 7.5, were added 3 µl of a 5 mg/ml solution of 2,4-dinitrobenzaldehyde or 4-N'-benzylamidobenzaldehyde in dimethyl sulfoxide and the reaction was allowed to proceed at 23 °C for 16 hours.

The 4-N'-benzylamidobenzaldehyde was made by adding under nitrogen 0.022 ml (0.2 mmole) of benzylamine to a solution of 49 mg (0.2 mmoles) of 4-carboxybenzaldehyde-N-hydroxysuccinimide ester (which had been prepared following Krachenbuhl et al., J. Exp. Med. 139, 208 - 223 (1974)) in 2 ml of dry N,N-dimethylformamide ("DMF") and allowing the reaction to proceed for 2.5 hours at 23 °C. After the 2.5 hour reaction, solvent was removed by rotary evaporation and the residual oil was taken up in 20 ml of ethyl acetate. The resulting solution was then washed successively with 10 ml of cold HCl, 10 ml of saturated NaHCO₃, and 10 ml of brine and finally dried over anhydrous MgSO₄ and concentrated under vacuum. The product was purified using preparative, thin-layer chromatography ("TLC") using 40 % ethyl acetate in hexane to afford 25 mg of 4-N'-benzylamidobenzaldehyde (yield: 53 %). The infrared spectrum of the product had peaks at 3315, 1704 and 1633 cm⁻¹. The ¹H NMR of the product in CDCl₃ at 350 MHz showed shifts of 10.05 (1H), 7.93 (4H), 7.35 (5H), and 4.65 (doublet, 2H) ppm. Elemental analysis of the product yielded the following result: Calculated: C (75.31 %), H (5.44 %), N (5.86 %); found: C(75.46 %), H(5.63 %), N(6.07 %).

The foregoing procedure for making the DNP and BAP derivatives of the oligonucleotides was carried out with (A) the oligonucleotides phosphorylated with ³²PO₄; (B) the oligonucleotides first phosphorylated with ³²PO₄ and then derivatized with imidazole, as described above; and (C) the oligonucleotides first phosphorylated with ³²PO₄ and then derivatized with hydrazine, carbohydrazide or adipic dihydrazide as described above. The reaction mixtures were analyzed by denaturing polyacrylamide gel electrophoresis ("PAGE") on a 20 % polyacrylamide gel with 8 M urea followed by autoradiography, following Maxam and Gilbert, Proc. Natl. Acad. Sci. (USA) 74, 560 - 564 (1977). As controls, the oligonucleotides derivatized at the 5'-carbon of the 5'-nucleotide with only ³²PO₄ or ³²P-phosphoimidazolide and dissolved at a concentration of 2.5 nanomoles/ml in 37.5 % (v/v) DMSO in 50 mM MOPS, pH 7.5, but not exposed to reactants to make the hydrazino and hydrazone-linker-containing derivatives were analyzed by PAGE.

In the PAGE analysis, only single hydrazone-linker-containing products could be found with the oligonucleotides derivatized with hydrazine, carbohydrazide or adipic dihydrazide. No evidence of hydrazone-linker-containing products was found with the oligonucleotides that, prior to reaction with 2,4-dinitrobenzaldehyde or 4-N'-benzylamidobenzaldehyde, were derivatized only with -PO₄⁻² or phosphoimidazolide. Surprisingly, with the reaction mixtures from reaction of the adipic dihydrazide-derivatized oligonucleotides with 4-N'-benzylamidobenzaldehyde as described above, complete disappearance of the hydrazino derivative was found whereas, in the reaction mixtures from the same reaction with 2,4-dinitrobenzaldehyde, some unreacted hydrazino derivative was obtained.

Hydrazone-linker-containing oligonucleotide derivatives were further analyzed after purification by analytical reverse phase HPLC. Thus, to 1 nanomole of oligonucleotide, derivatized as described above with -(PO₃)NHNH₂ or -(PO₃)NH(NH)(CO)(NH)NH₂, in 1 ml of 50 mM MOPS, pH 7.5, were added 25 µl of a 5 mg/ml acetonitrile solution of 2,4-dinitrobenzaldehyde or 4-N'-benzylamidobenzaldehyde, and the reaction was allowed to continue for 16 hours at 23 °C. Hydrazone-linker-containing oligonucleotide was isolated from the crude reaction mixture by LiCl/ethanol precipitation. Purification of the hydrazone-derivatized oligonucleotide was then accomplished using a Vydac analytical reverse-phase column (C-8, 0.46 x 25 cm)(VYDAC-The Seps. Group, Inc., Hesperia, California, USA) with a gradient of 0 - 35 % acetonitrile in 0.1 M triethylammonium acetate, pH 6.8, over 45 minutes. The fractions with hydrazone-derivatized oligonucleotide were then concentrated to dryness in a Savant spin-vaccuum system (Savant Instruments, Inc., Farmingdale, New York, USA) and the resulting pellet was then taken up in 50 mM MOPS, pH 7.5. The absorption spectra of the resulting solutions were recorded between 230 and 450 nm using a Beckman DU-40 UV-VIS spectrophotometer (Beckmann Instruments, Inc., Fullerton, California, USA). The 2,4-dinitrophenyl group absorbs at 370 nm while the 4-N'-benzylamidophenyl group absorbs at 320 nm. Analysis of the elution profiles of the hydrazone-linker-containing oligonucleotides and oligonucleotides derivatized with only 5'-PO₄ established that only one detectable hydrazone group was linked to each oligonucleotide molecule.

Further, measurements establishing that the hydrazino- and hydrazone-derivatized oligonucleotides were not affected by alkaline phosphatase under conditions which resulted in dephosphorylation of the 5'-phosphate-containing oligonucleotide established that the single hydrazino group or hydrazone was linked to the 5'-carbon of the 5'-terminal nucleotide of the oligonucleotide.

### EXAMPLE 2

### DERIVATIZATION OF CALF INTESTINE ALKALINE PHOSPHATASE TO HAVE FREE ALDEHYDE GROUPS

Calf intestine alkaline phosphatase (enzyme immunoassay grade) was obtained from Boehringer Mannheim Biochemicals, Inc. (Indianapolis, Indiana, U.S.A.).

2.86 x 10⁻⁸ mol of the enzyme in 0.4 ml of a solution of 3 M NaCl, 0.1 mM MgCl₂, 1 mM ZnCl₂, and 30 mM triethanolamine, pH 7.6, was dialyzed, over 2 hours, against three changes of 30 ml each of 0.1 M NaHCO₃, 3 M NaCl, 0.02 % NaN₃, pH 8.5, using a collodion bag (mol. wt. cutoff: 25 kd)(Schleicher and Schuell, Inc., Keene, New Hampshire, USA) at 4 °C. Thereafter, a 7.5-fold or 70-fold molar excess of p-carboxybenzaldehyde-N-hydroxy-succinimide ester, in a 50 mM solution in acetonitrile, was added to the enzyme solution and the reaction allowed to proceed for 30 minutes at 23 °C. After the 30 minutes, excess reagent was removed by dialysis, over 2 hours, against 3 changes of 30 ml each of 50 mM MOPS, 0.1 M NaCl, pH 7.5, using a collodion bag at 4 °C.

When a 7.5-fold excess of p-carboxybenzaldehyde-N-hydroxysuccinimide ester was employed, an average of 1.1 aldehyde groups were introduced per enzyme molecule. When a 70-fold excess of p-carboxybenzaldehyde-N-hydroxysuccinimide ester was employed, an average of 3.6 aldehyde groups were introduced per enzyme molecule. These determinations were from measurements of the absorbances of the modified enzymes at 258 nm and 280 nm.

The enzyme is also modified in substantially the same way but employing the N-hydroxysuccinimide esters of formulas H(CO)(CH₂)₂(C₆H₄)(CO)ON(C₄H₄O₂) and (C₄H₄O₂)NO(CO)(CH₂)₂(C₆H₄)(CO)H in place of p-carboxybenzaldehyde-N-hydroxysuccinimide ester.

### EXAMPLE 3

### PREPARATION AND PROPERTIES OF CONJUGATES OF ALDEHYDE-GROUP-DERIVATIZED CALF INTESTINE ALKALINE PHOSPHATASE WITH 5'-HYDRAZINO-DERIVATIZED OLIGONUCLEOTIDES

8 nmol of pelleted oligonucleotide 85-133, derivatized as in Example 1 to have a group of formula -(PO₃)(NH)(NH)(CO)(NH)NH₂ covalently joined to the 5'-carbon of the 5'-terminal nucleotide was taken up in a 3.5 molar excess of a 70 µM solution of aldehyde-group derivatized calf intestine alkaline phosphatase in 50 mM MOPS, 0.1 M NaCl, pH 7.5. The aldehyde-group-derivatized enzyme had been prepared as in Example 2 with a 70-fold molar excess of p-carboxybenzaldehyde-N-hydroxysuccinimide ester. The reaction of derivatized oligonucleotide with derivatized enzyme was allowed to proceed at 23 °C for 16 hours.

It is noteworthy that hyrazino-derivatized oligonucleotide did not need to be separated from underivatized, phosphorylated oligonucleotide prior to reaction with aldehyde-group-derivatized enzyme because, as indicated by the model studies described above in Example 1, the aldehyde function reacts exclusively with the free hydrazino-group joined to the 5'-carbon of the 5'-terminal nucleotide of the oligonucleotide. No detectable reaction occurs between 5'-phosphorylated oligonucleotide (or oligonucleotide with unmodified hydroxyl at the 5'-carbon of the 5'-nucleotide) and aldehyde-derivatized enzyme.

Gel filtration in a Biorad P-100 column (1.5 x 65 cm)(Biorad, Inc., Richmond, California, USA) at 4 °C using 0.05 M Tris, pH 8.5, as eluant separated unreacted oligonucleotide from enzyme-oligonucleotide conjugate and unreacted enzyme. From measurements of radioactivity in the unreacted, hydrazino-group-derivatized oligonucleotide in comparison with that in the oligonucleotide-enzyme conjugate, when ³²-labeled oligonucleotide was employed in preparing the conjugates, 80 - 85 % of hydrazino-group-derivatized oligonucleotide was found to have been conjugated to enzyme in the conjugation reaction.

Enzyme fractions from the gel filtration were pooled and applied to a DEAE-cellulose column (1 x 7.2 cm)(DE-52, Whatman, Inc., Clifton, New Jersey, USA) equilibrated with 0.05 M Tris, pH 8.5, at 23 °C. The column was washed with 0.1 M Tris, pH 8.5 (15 ml), followed by a 40 ml salt gradient of 0 - 0.2 M NaCl in 0.1 M Tris, pH 8.5, to elute free alkaline phosphatase.

A stepwise isocratic elution (0.2 M NaCl, 0.1 M Tris, pH 8.5, 40 ml, followed by 0.5 M NaCl, 0.1 M Tris, pH 8.5, 20 ml) on DEAE-cellulose (1 x 7.2 cm, DE-52) achieved the separation of 1:1 oligonucleotide-enzyme conjugate from 2:1 oligonucleotide-enzyme conjugate. From measurements of radioactivity due to the two conjugates, when they were prepared with ³²P-labeled oligonucleotide, it was found that the 1:1 conjugate and 2:1 conjugate were made in approximately equimolar amounts.

To complete the preparations of the conjugates, for each of the two conjugates, fractions with the conjugate were pooled, concentrated using centriprep-30 concentrators (Amicon Corp., Danvers, Massachusetts, USA) and stored in 0.1 M Tris, 0.1 M NaCl, pH 8.5, at 4 °C.

The conjugates were assayed for enzymatic activity colorimetrically employing p-nitrophenylphosphate as substrate. Thus, the hydrolysis of this substrate was followed at 410 nm and 23 °C in 0.1 M Tris, 0.1 M NaCl, 0.01 M MgCl₂, pH 9.5, with an initial concentration of substrate of 0.1 mM. It was found that the activity of the enzyme in the conjugates was 80 - 85 % of that of the free enzyme.

The conjugates have been found to be extremely stable. They lose none of their sensitivity in hybridization assays when stored for at least several months as described above at 4 °C.

By carrying out Lowry protein determinations (calibrated using dilutions of aldehyde-group-derivatized alkaline phosphatase whose concentrations were determined by amino acid analysis using a Beckmann System 6300 amino acid analyzer after 24 hour hydrolysis at 110 °C) on samples of the two conjugates which had equivalent amounts of radioactivity (due to ³²P of the linker), it was found that the "low salt" conjugate (i.e., the one eluted from DEAE cellulose at 0.2 M NaCl, 0.1 M Tris, pH 8.5, as described above) had twice the amount of protein as the "high salt" conjugate (i.e., the one eluted from DEAE cellulose at 0.5 M NaCl, 0.1 M Tris, pH 8.5, as described above), thereby establishing that the "low salt" conjugate was the 1:1 conjugate and the "high salt" conjugate the 2:1 conjugate. Both of the conjugates co-migrated in gel filtration on Biorad P-200 (Trade Mark) (Biorad, Inc.) and Sephacryl S-300 (Trade Mark) (Pharmacia, Inc., Piscataway, New Jersey, USA), consistently with their similar, calculated molecular weights of about 150 kd and 160 kd, respectively. Further, the "high salt" conjugate had higher electrophoretic mobility than the "low salt" on a 2 % agarose gel, consistently with the higher charge/mass balance of the "high salt" species.

When the conjugation reaction was carried out as described above but with a 5-fold or a 10-fold molar excess (in place of a 3.5-fold molar excess) of aldehyde-group-derivatized enzyme that had been prepared with a 7.5-fold molar excess (in place of a 70-fold molar excess) of p-carboxybenzaldehyde-N-hydroxysuccinimide ester (and, consequently, had an average of 1.1 (in place of 3.6) aldehyde groups per enzyme molecule), 40 - 45 % of hydrazino-group-derivatized oligonucleotide was conjugated to enzyme and the ratio of 1:1 conjugate to total conjugate was about 0.8 when 5-fold molar excess of aldehyde-group derivatized enzyme was used and 50 - 55 % of hydrazino-group-derivatized oligonucleotide was conjugated to enzyme and the ratio of 1:1 conjugate to total conjugate was about 0.9 when 10-fold molar excess of aldehyde-group-derivatized enzyme was used.

Conjugates described above, with linkers which comprise an hydrazone group, have been converted as follows to conjugates wherein the hydrazone group of the linker is converted to an hydrazide group (-(NH)(NH)(CH₂)-): 500 µl of a 2 µM solution of conjugate with hydrazone-group-containing linker in 0.1 M Tris, 0.1 M NaCl, pH 8.5, was dialyzed at 4 °C over 2 hours against 3 changes of 30 ml each change of 0.1 M MES (2-(N-morpholino)ethanesulfonic acid), pH 6.00, and then 0.4 M sodium cyanoborohydride was added to bring the concentration of the cyanoborohydride to 0.02 M. The reaction was allowed to proceed at 23 °C for 16 hours and then the solution was exhaustively dialyzed against 5 or 6 changes (30 ml each) of 0.1 M Tris, 0.1 M NaCl, pH 8.5, at 4 °C over 4 hours. Conjugates with the hydrazone reduced as described to the hydrazide show no loss in activity of the enzyme.

Conjugates, with linkers which comprise an hydrazone group and linkers which comprise an hydrazide group, are provided in substantially the same way as described in this Example employing calf intestine alkaline phosphatase derivatized as described in Example 2 with 50-fold molar excesses of the N-hydroxysuccinimide esters of formulas H(CO)(CH₂)₂(C₆H₄)(CO)ON(C₄H₄O₂) and (C₄H₄O₂)NO(CO)(CH₂)₂(C₆H₄)(CO)H and oligonucleotide derivatized as described in Example 1 at the 5'-carbon of the 5'-terminal nucleotide with a moiety of formula -(PO₃)(NH)(NH)(CO)(NH)NH₂ and employing calf intestine alkaline phosphatase derivatized as described in Example 2 with a 50-fold molar excess of p-carboxybenzaldehyde-N-hydroxysuccinimide ester and oligonucleotide derivatized as described in Example 1 at the 5'-carbon of the 5'-terminal nucleotide with a moiety of formula -(PO₃)(NH)NH₂ or -(PO₃)(NH)(NH)(CO)(CH₂)₄(NH)NH₂.

Conjugates are also made, as described in this Example, with oligonucleotide 87-416 or any other single-stranded nucleic acid of between about 8 and about 150 bases in length.

### EXAMPLE 4

### HORSERADISH PEROXIDASE DERIVATIZED TO HAVE FREE ALDEHYDE GROUPS

Horseradish peroxidase is a glycoprotein and, consequently, has covalently associated with it sugar moieties which have vicinal hydroxyl groups which are susceptible to oxidation to aldehyde groups using periodate as the oxidizing agent.

Thus, Type VII horseradish peroxidase was obtained from Sigma Chemical Co. (St. Louis, Missouri, U.S.A.). To 1 mg (2.5 x 10⁻⁸ mol) of horseradish peroxidase dissolved in 0.5 ml of water was added 0.1 ml of an 0.2 M aqueous sodium periodate solution. The oxidation reaction was allowed to proceed for 20 minutes at 23 °C and then excess periodate was removed by dialysis against 30 ml of 1 mM sodium acetate, pH 4.5, at 4 °C using a collodion bag (molecular weight cut-off 25 kd). This was followed by dialysis over 2 hours against 3 changes of 30 ml each of 0.1 M MOPS, 0.1 M NaCl, pH 7.5, at 4 °C using a collodion bag, prior to use of the aldehyde-group-containing enzyme in a conjugation reaction.

Horseradish peroxidase (type VII, Sigma Chemical Corp.) was also derivatized to be covalently joined to free aldehyde group(s) by treatment with a 50-fold molar excess of p-carboxybenzaldehyde-N-hydroxysuccinimide ester, as described in Example 2 for calf intestine alkaline phosphatase.

The enzymatic activities of the horseradish peroxidases modified to have free aldehyde groups were assayed by a standard technique employing for color development an aqueous solution of 0.5 mg/ml of 3,3'-diaminobenzidine hydrochloride ("DAB"), 0.05 M Tris, 0.04 % NiCl₂, pH 7.6. 1 µl of an approximately 0.04 µmol/ml solution of enzyme to be assayed was added to 20 ml of the color development solution. With unmodified enzyme as well as the enzyme modified by both methods described in this example, a blue-black precipitate developed, indicating that the enzymes were catalytically active. Quantitative analysis of the rate of color development indicated that the enzyme modified with periodate was at least 100-fold less active than the unmodified enzyme and that the enzyme modified by reaction with the 50-fold molar excess of p-carboxybenzaldehyde-N-hydroxysuccinimide ester was 70 - 75 % as active as the unmodified enzyme.

### EXAMPLE 5

### PREPARATION AND PROPERTIES OF CONJUGATES OF ALDEHYDE-GROUP-DERIVATIZED HORSERADISH PEROXIDASE WITH 5'-HYDRAZINO-DERIVATIZED OLIGONUCLEOTIDES

4 nmol of oligonucleotide 85-133, derivatized according to Example 1 to have a group of formula -(PO₃)(NH)(NH)(CO)(NH)NH₂ joined to the 5'-carbon of the 5'-terminal nucleotide, in pellet form were taken up in 500 µl of solution of horseradish peroxidase modified with periodate in accordance with Example 4 or horseradish peroxidase modified by reaction with p-carboxybenzaldehyde-N-hydroxysuccinimide ester, in accordance with Example 4, dissolved at 40 nmol/ml in 0.1 M MOPS, 0.1 M NaCl, pH 7.5. The conjugation reactions were allowed to proceed for 16 hours at 23 °C.

The oligonucleotide-enzyme conjugate and unreacted enzyme from a conjugation reaction were separated from unreacted oligonucleotide by gel filtration on a Sephadex G-75 (Trade Mark) column (Pharmacia, Inc.)(1.5 x 47 cm) at 4 °C using 0.05 M Tris, pH 8.5, as eluant. Pooled enzyme fractions were then applied to a DEAE cellulose column (DE-52, Whatman, Inc.)(1 x 7.2 cm) that had been equilibrated with 0.05 M Tris, pH 8.5, at 23 °C. Step washes with 0.1 M Tris, pH 8.5; 0.1 M Tris, 0.1 M NaCl, pH 8.5; and 0.1 M Tris, 0.2 M NaCl, pH 8.5, were used to elute unreacted enzyme. Elution of oligonucleotide-enzyme conjugate was then achieved using 0.1 M Tris, 0.5 M NaCl, pH 8.5.

The ratio of oligonucleotide to enzyme in the conjugate has not been determined, although it is presumed, based on analogy with the "high salt" alkaline phosphatase conjugate described in Example 3, that the ratio is 2:1.

Measurements of the efficiencies of the conjugation reactions, by employing the methods described in Example 3, showed that 55 - 60 % of the oligonucleotide was incorporated into conjugate when the periodate-modified enzyme was employed and 35 - 40 % of the oligonucleotide was incorporated when the p-carboxybenzaldehyde-N-hydroxysuccinimide ester-modified enzyme was used.

Assay of enzymatic activity of the enzyme in the conjugates by the method described in Example 4, assuming that the ratio of molecules of oligonucleotide to molecules of enzyme in the conjugates is 2:1, showed that the conjugation reaction did not significantly change the enzymatic activity from that of the enzyme modified to have free aldehyde groups. The enzyme in both conjugates remained catalytically active. The enzyme in the conjugate made with periodate-modified enzyme was more than 100 times less active than unmodified enzyme. The enzyme in the conjugate made with p-carboxybenzaldehyde-N-hydroxysuccinimide ester-modified enzyme retained 70 - 75 % of the activity of the unmodified enzyme.

Conjugates were also prepared as described in this Example with oligonucleotide 87-416, with substantially the same results.

Conjugates are also prepared as described in this example with the oligonucleotides modified to have a group of formula -(PO₃)(NH)NH₂ or -(PO₃)(NH)(NH)(CO)(CH₂)₄(CO)(NH)NH₂ covalently joined to the 5'-carbon of the 5'-terminal nucleotide.

### EXAMPLE 6

### SANDWICH HYBRIDIZATION ASSAY USING CALF INTESTINE ALKALINE PHOSPHATASE-OLIGONUCLEOTIDE CONJUGATE AS DETECTION PROBE

"OligoBead™" brand oligonucleotide-derivatized macroporous support (Siska Diagnostics, Inc., La Jolla, California, USA) ("beads") was prepared as described by Ghosh and Musso, Nucl. Acids Research 15, 5353 (1987), by derivatizing cyanogen bromide-activated "Sephacryl S-500 (Trade Mark)" brand dextran macroporous support material (Pharmacia Inc., Piscataway, New Jersey, USA) with 6-aminocaproic acid to provide carboxylate groups covalently joined to the support material and then derivatizing the carboxylate-derivatized support with the oligonucleotide ("capture probe") of sequence:
5'-TGCTGCTATGCCTCATCTTCTTATTGGTT-3',
derivatized, at the 5'-carbon of the 5'-terminal nucleotide, with the moiety of formula -(PO₃)(NH)(CH₂)₆NH₂. See also PCT International Publication No. WO88/01302.

The capture probe has the sequence complementary to that of oligonucleotide 87-416, described in Example 1.

50 mg of the "OligoBead (Trade Mark)" beads with capture probe were prehybridized by soaking for 15 minutes at 37 °C in 0.75 ml of 5 x SSC (0.75 M NaCl, 0.075 M sodium citrate, pH 7.0) , 0.1% sodium dodecyl sulfate (SDS), 10% dextran sulfate (Pharmacia, Inc.), 1 mg/ml sonicated salmon sperm DNA, and 1 mg/ml bovine serum albumin fraction V (Boehringer Mannheim Biochemicals, Inc.).

M13 phage were prepared by a standard technique to have a genome which comprised one strand of the EcoRI fragment of plasmid pTBO61B coding an hepatitis B virus surface antigen. The strand of the EcoRI fragment that was in the phage genome was the strand that comprises a segment with the sequence of oligonucleotide 87-416. The phage genome, which is a single-stranded DNA, was the nucleic acid analyte in the assay described in this example. This analyte comprises a target segment which has the sequence complementary to that of oligonucleotide 85-133, described in Example 1.

Either 5 fmol or 50 fmol of the single-stranded M13 genome and a five-fold molar excess (relative to M13 genome target) of calf intestine alkaline phosphatase-oligonucleotide 85-133 conjugate, wherein the enzyme and oligonucleotide were present in a 1:1 ratio and were covalently joined by a linker of formula -(PO₃)(NH)(NH)(CO)(NH)N=CH(C₆H₄)(CO)- and which was prepared by the method of Example 3, were combined in a 50 µl solution of 5 x SSC, 5% dextran sulfate, and 1 mg/ml of bovine serum albumin fraction V and heated for 5 minutes at 65°C. The target DNA and enzyme-oligonucleotide conjugate were then permitted to hybridize for 30 minutes at 42°C. (When target is present at less than about 40 fmol/ml, this solution hybridization is continued for at least two hours.)

After the pre-hybridization, the beads were pelleted by centrifugation with a benchtop centrifuge from the prehybridization solution and the supernatant was removed by aspiration with a pipette and discarded. Then the beads were taken up in 200 µl of 5 x SSC, 0.1% SDS, 10% dextran sulfate, and 1 mg/ml bovine serum albumin fraction V.

Then the 50 µl solution in which target DNA and enzyme-oligonucleotide conjugate were hybridized was combined with the 200 µl of solution with the pre-hybridized beads and the resulting solution was incubated for 90 minutes at 42 °C for hybridization of target (hybridized to detection probe) to capture probe on the beads.

Then the beads were pelleted by centrifugation with a benchtop centrifuge, the supernatant was removed by aspiration with a pipette and discarded, and, finally, the beads were washed three times with 1 ml of 2 x SSC (0.3 M NaCl, 0.03 M sodium citrate, pH 7.0) each time by soaking for 10 minutes at 37 °C.

Hybridization of the enzyme-oligonucleotide conjugate detection probe to target DNA captured by the capture probe covalently affixed to the beads was determined by color development. After the third wash in 2 x SSC, the pelleted beads were rinsed with 1.5 ml of a developing buffer (100 mM NaCl, 10 mM MgCl₂, and 100 mM Tris, pH 9.5) and then, after pelleting the beads by centrifugation in a tabletop centrifuge and removal of the buffer by aspiration with a pipette, color development was initiated by adding to the beads 1 ml of developing buffer which included 0.1 mM p-nitrophenyl phosphate and vortexing the mixture to suspend the beads in the buffer. Color development was allowed to proceed for 1.25 hours at 23 °C. After the 1.25 hours, the support material was pelleted by centrifugation and the absorbance of the supernatant was measured at 410 nm.

The absorbances measured at 410 nm with the samples described above were compared with those measured with controls. The control runs were the same as those described above except that, in place of the beads derivatized with oligonucleotide 85-133, beads were used that were derivatized with an oligonucleotide with a sequence such that no hybridization between the oligonucleotide and target segment would have been expected under the stringencies of the hybridization and washes. For each of the samples, the absorbance was at least twice that for the corresponding control.

Thus, the calf intestine alkaline phosphatase-oligonucleotide 1:1 conjugate is capable of detecting fewer than about 10⁹ molecules of target in the above-described sandwich assay system.

### EXAMPLE 7

### NITROCELLULOSE FILTER-BASED HYBRIDIZATION ASSAY USING ENZYME-OLIGONUCLEOTIDE CONJUGATES AS DETECTION PROBES

Conjugates of calf intestine alkaline phosphatase (both "low salt" and "high salt") and horseradish peroxidase joined to oligonucleotide 85-133 by the linker of formula -(PO₃)(NH)(NH)(CO)(NH)N=CH(C₆H₄)(CO)- and prepared as described in examples 3 and 4 were employed as detection probes in filter-based assays for plasmid pTB061B.

10 ng, 1 ng, 100 pg, 50 pg, 25 pg, 10 pg and 1 pg of plasmid pTB061B, 10 µg of human DNA, 1 µg of E. coli DNA, and 50 ng of pBR322 were denatured under alkaline conditions at 65°C (0.2 M NaOH, 15 minutes), neutralized with 2 M ammonium acetate, and then, using a Schleicher and Schuell (Keene, New Hampshire, USA) Minifold II slot-blot system, slot-blotted onto a nitrocellulose membrane which had been pretreated with 10 x SSC. The filter was then baked at 80 °C under vacuum and pre-hybridized by being incubated for 10 minutes at 50 °C in an heat-sealable plastic bag with 3 ml of a hybridization buffer (5 x SSC, 5 mg/ml bovine serum albumin fraction v, 5 mg/ml of polyvinyl pyrrolidone (average molecular weight 40,000 d, Sigma Chemical Company, Inc., St. Louis, Missouri, U.S.A.), and 0.1% SDS.

After the filters were prehybridized, they were hybridized in the bag for 1 hour at 50 °C with conjugate at 2 µg/ml in the hybridization buffer.

After the hybridization, the filter was removed from the bag and washed three times, for three minutes per wash, in 1 x SSC, 0.1% SDS at 23 °C. The filter was then subjected to a stringency wash for one minute in 1 x SSC, 0.1% SDS at 50°C.

Then, for assays with the alkaline phosphatase conjugates, the filter was rinsed three times with developing buffer (0.1 M Tris, 0.1 M NaCl, and 10 mM MgCl₂, pH 9.5) and then placed face up in 5 ml of developing buffer to which had been added nitrotetrazolium blue to 0.33 mg/ml, 5-bromo-4-chloro-3-indolyl phosphate to 0.16 mg/ml and N,N-dimethyformamide(DMF) to 0.33 % (v/v). The filter was allowed to sit in the solution in the dark for 1 hour at 23 °C in order for color development to occur.

For assays with the horseradish peroxidase conjugate, after the stringency wash the filter was washed with 0.05 M Tris, pH 7.6, and then developed by being placed face up in 5 ml of a solution of 0.5 mg/ml 3,3'-diaminobenzidine hydrochloride, 0.05 M Tris, pH 7.6, containing 0.04 % NiCl₂ and being allowed to sit in the solution for 1 hour at 23 °C.

Neither the alkaline phosphatase conjugates nor the horseradish peroxidase conjugate yielded a visually observable signal in the assays of human DNA, E. coli DNA or pBR322.

The alkaline phosphatase conjugates both yielded clearly visually observable signals reproducibly with 25 pg of pTB061B and sometimes with 10 pg of pTB061B. Thus, it should be possible to detect as few as 3 attomol of a nucleic acid analyte with an alkaline phosphatase-oligonucleotide conjugate probe in accordance with the present invention in a filter-based hybridization assay similar to that described in this example.

Using ³²P-labeled oligonucleotide 85-133 in the hybridization assay described in this example, 18 hours of autoradiography was required to detect 25 pg of pTB061B.

The horseradish peroxidase conjugate yielded a clearly visually observable signal with 1 ng of pTB061B. Thus, it should be possible to detect fewer than 0.3 femtomol of a nucleic acid analyte with an horseradish peroxidase-oligonucleotide conjugate probe in accordance with the present invention (wherein the peroxidase is modified at free amino group(s) to be covalently joined to a free aldehyde group) in a filter-based hybridization assay similar to that described in this example.

It may be possible to increase this sensitivity with substrates for the peroxidase other than diaminobenzidine/nickel chloride, such as the chemiluminescent assay employing luminol and parahydroxycinnamic acid in sodium borate buffer, pH 8.3, and reading signal with a luminometer, as described by Urdea et al., Gene 61, 253 -264 (1987). With this chemiluminescent assay of Urdea et al., luminescent product of the enzymatic reaction is released into solution; thus, rather than develop bands directly on the filter, bands are cut from the filter and suspended in the developing solution for signal development.

### EXAMPLE 8

### CALF INTESTINE ALKALINE PHOSPHATASE-OLIGONUCLEOTIDE CONJUGATE PROBE IN SOUTHERN BLOT ANALYSIS

Plasmid pTB061B was digested to completion with EcoRI and 3-fold serial dilutions of cleaved plasmid in 0.04 M Tris, 1 mM EDTA, pH 7.8, 1 µg/ml ethidium bromide (i.e., 100 ng, 33.3 ng, 11.1 ng, 3.7 ng, 1.2 ng, 410 pg, 137 pg, 45 pg, 15 pg, 5 pg, and 1.7 pg of plasmid) were electrophoresed through separate lanes of a 1.5 % agarose gel. The gel was photographed under UV light, and then the DNA fragments were transferred to a nitrocellulose filter following the procedure of Southern, J. Mol. Biol. 98, 503 - 517 (1975). The filter was then developed following the procedure described in Example 7 using 2 µg/ml of alkaline phosphatase-oligonucleotide 85-133 "high salt" conjugate probe and 1 hour of color development. With the ethidium bromide staining, only the pBR322 portion of pTB061B could be detected, and then in only the 100 ng and 33.3 ng lanes. In contrast, with the conjugate probe for DNA transferred to a filter, only the surface antigen coding portion of pTB061B could be detected but in all lanes down to the 410 pg lane, where color was clearly visually detectable.

### EXAMPLE 9

### DERIVATIZATION OF UREASE TO BE COVALENTLY JOINED TO FREE ALDEHYDE GROUPS AND PREPARATION OF UREASE-OLIGONUCLEOTIDE CONJUGATE PROBE

Jack bean urease (type VII) was obtained from Sigma Chemical Co., St. Louis, Missouri, USA.

25 nmol of the urease in 0.25 ml of water was dialyzed at 4 °C over 2 hours against three changes of 30 ml each of 0.1 M NaHCO₃, 3 M NaCl, pH 8.5. Then a 50-fold molar excess of p-carboxybenzaldehyde-N-hydroxysuccinimide ester in 30 µl of acetonitrile was added to the enzyme solution and the reaction was allowed to proceed for 30 minutes at 23 °C. Excess p-carboxybenzaldehyde-N-hydroxysuccinimide ester was then removed by dialysis at 4 °C over 2 hours against 3 changes of 30 ml each of 0.05 M MOPS, 0.1 M NaCl, pH 7.5.

4 nmol of oligonucleotide 85-133, derivatized at the 5'-carbon of the 5'-terminal nucleotide with the moiety of formula -(PO₃)(NH)(NH)(CO)(NH)NH₂ and in pellet form, was taken up in 200 µl of solution of the urease, modified to be covalently joined to free aldehyde groups and dissolved in 0.05 M MOPS, 0.1 M NaCl, pH 7.5, as described above in this example. The conjugation reaction was allowed to proceed for 16 hours at 23 °C. Then urease-oligonucleotide conjugate and unreacted urease were separated from unreacted oligonucleotide by gel filtration through a Biorad P-100 column (1.5 x 65 cm) at 4 °C using 0.05 M Tris, pH 8.5, as eluant. The conjugate was then separated from unreacted enzyme by ion-exchange chromatography of the enzyme- and conjugate-containing fractions from the gel filtration over a DEAE cellulose (DE-52, Whatman) column (1 x 7.2 cm) eluted with 15 ml of 0.05 M Tris, pH 8.5; 15 ml of 0.1 M Tris, pH 8.5; 20 ml of 0.1 M Tris, 0.1 M NaCl, pH 8.5; 40 ml of 0.1 M Tris, 0.2 M NaCl, pH 8.5; and 40 ml of 0.1 M Tris, 0.5 M NaCl, pH 8.5. The urease-oligonucleotide conjugate eluted with the 0.1 M Tris, 0.5 M NaCl, pH 8.5. Finally, the conjugate-containing fraction was desalted and concentrated to 1 ml using a Centricon 30™ microconcentrator (molecular weight cutoff: 30 kd)(Amicon Corp., Danvers, Massachusetts, USA).

### EXAMPLE 10

### DERIVATIZATION OF CARBONIC ANHYDRASE TO BE COVALENTLY JOINED TO FREE ALDEHYDE GROUPS AND PREPARATION OF CARBONIC ANHYDRASE-OLIGONUCLEOTIDE CONJUGATE PROBE

Bovine erythrocyte carbonic anhydrase was obtained from Cooper Biomedical, Inc., Malvern, Pennsylvania, USA. The enzyme was derivatized, using p-carboxybenzaldehyde-N-hydroxysuccinimide ester, to be covalently joined to free aldehyde groups by following the procedure described in Example 9 for urease. The derivatization process began with 25 nmol of the enzyme in a 0.1 µmol/ml solution and yielded 250 µl of solution of desired, aldehyde-group-derivatized enzyme at about 0.1 µmol/ml in 0.05 M MOPS, 0.1 M NaCl, pH 7.5.

4 nmol of oligonucleotide 85-133, derivatized at the 5'-carbon of the 5'-terminal nucleotide with the moiety of formula -(PO₃)(NH)(NH)(CO)(NH)NH₂ and in pellet form, was taken up in 200 µl of the solution of aldehyde-group-derivatized carbonic anhydrase in 0.05 M MOPS, 0.1 M NaCl, pH 7.5, and the conjugation reaction between the oligonucleotide and enzyme was allowed to proceed for 16 hours at 23 °C. Then carbonic anhydrase-oligonucleotide conjugate and unreacted carbonic anhydrase were separated from unreacted oligonucleotide by gel filtration at 4 °C on a Sephadex G-75 (Trade Mark) column, as described in Example 5, using 0.05 M Tris, pH 8.5, as eluant. Then ion exchange chromatography of the free enzyme-containing and conjugate-containing fractions from the gel filtration was carried out as described in Example 9 to separate unreacted enzyme from conjugate. Finally, the conjugate fractions were desalted and concentrated as described in Example 9 using a Centricon 30 (Trade Mark) microconcentrator (molecular weight cutoff: 30 kd).

The conjugate probe is detected through the colored product generated upon the hydrolysis, catalyzed by the carbonic anhydrase, of p-nitrophenyl acetate. The enzyme in the probe has been found to be catalytically active in such hydrolysis.

## Claims

1. A nucleic acid probe which comprises a single-stranded nucleic acid and a catalytically active enzyme, wherein said nucleic acid comprises a segment of at least 20 nucleotides in the sequence complementary to the sequence of the target segment of said probe; wherein said nucleic acid and said enzyme are linked by a linker of a formula selected from the group consisting of formula I
-(PO₃)NH(R₁)N=CH(R₂)(CO)- I
and formula II
-(PO₃)NH(R₁)NH(CH₂)(R₂)(CO)- II
wherein R₁ is selected from the group consisting of a bond between the nitrogens, (NH)(CO)(NH), 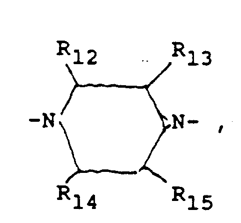 and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein R₂ is selected from the group consisting of alkylene of 1 to 20 carbon atoms and arylene of formula: 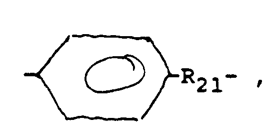 wherein R₂₁ is alkylene of 0 to 10 carbon atoms; wherein the -(PO₃)- group is bonded to the 5'-carbon of the 5'-nucleotide of the nucleic acid; and wherein the carbons of the carbonyl groups at the right-hand sides of figures I and II are bonded to the nitrogen of a free amino group of the enzyme.

2. A nucleic acid probe according to Claim 1 wherein the nucleic acid consists of 20-150 nucleotides in the sequence complementary to the sequence of the target segment; the catalytically active enzyme is selected from the group consisting of phosphatases, peroxidases, beta-galactosidases, ureases, carbonic anhydrases and luciferases; R₁ is selected from the group consisting of a bond between the nitrogens, (NH)(CO)(NH) and (NH)(CO)(CH₂)ₙ(CO)(NH), wherein n is is 0 to 10; and R₂ is selected from the group consisting of p-phenylene and (CH₂)ₘ, wherein m is 1 to 10.

3. A nucleic acid probe according to Claim 2 wherein the nucleic acid is 25-50 nucleotides in length; R₁ is selected from the group consisting of a bond between the nitrogens, (NH)(CO)(NH) and (NH)(CO)(CH₂)₄(CO)(NH); and R₂ is p-phenylene.

4. A nucleic acid probe according to any one of claims 1 to 3 wherein the enzyme is selected from the group consisting of calf intestine alkaline phosphatase, E. coli alkaline phosphatase, horseradish peroxidase, E. coli betagalactosidase, jack bean urease, bovine erythrocyte carbonic anhydrase, P. fischeri luciferase and firefly luciferase.

5. A nucleic acid probe which comprises a single-stranded nucleic acid and a catalytically active enzyme, wherein said nucleic acid comprises a segment of at least 20 nucleotides in the sequence complementary to the sequence of the target segment of said probe; wherein said enzyme is a glycoprotein that has been oxidized by treatment with periodate; wherein said nucleic acid and said enzyme are linked by a linker of a formula selected from the group consisting of formula IX
-(PO₃)NH(R₁)N= IX
and formula X
-(PO₃)NH(R₁)NH- X
wherein R₁ is selected from the group consisting of a bond between the nitrogens, (NH)(CO)(NH), 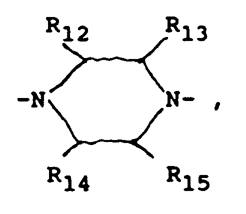 and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein the -(PO₃)- group is bonded to the 5'-carbon of the 5'-nucleotide of the nucleic acid; and wherein the -N= at the right-hand side of formula IX and the nitrogen of the -NH- group at the right-hand side of formula X are bonded to a carbon of a sugar residue of the enzyme, that was the carbon of a carbonyl group formed in the treatment of the enzyme with periodate.

6. A nucleic acid probe according to Claim 5 wherein the nucleic acid consists of 20-150 nucleotides in the sequence complementary to the sequence of the target segment; the catalytically active enzyme is an eukaryotic peroxidase, e.g. horseradish peroxidase; and R₁ is selected from the group consisting of a bond between the nitrogens, (NH)(CO)(NH) and NH)(CO)(CH₂)ₙ(CO)(NH), wherein n is 0 to 10.

7. A nucleic acid probe according to Claim 6 wherein the nucleic acid is 25-50 nucleotides in length; and R₁ is selected from the group consisting of a bond between the nitrogens, (NH)(CO)(NH) and (NH)(CO)(CH₂)₄(CO)(NH).

8. A single-stranded nucleic acid which is an intermediate for making a nucleic acid probe for detecting a target segment, comprises a segment of at least 20 nucleotides in the sequence complementary to that of said target segment, and is derivatized with a moiety of a formula XIV
-(PO₃)(NH)(R₁)NH₂ XIV
wherein R₁ is selected from the group consisting of a bond between the nitrogens, (NH)(CO)(NH), 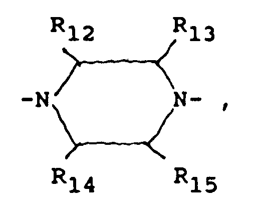 and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; and wherein the -(PO₃)- group is bonded to the 5'-carbon of the 5'-nucleotide of the nucleic acid.

9. A nucleic acid according to Claim 8 wherein the nucleic acid consists of 20-150 nucleotides in the sequence complementary to the sequence of the target segment; and R₁ is selected from the group consisting of a bond between the nitrogens, (NH)(CO)(NH) and (NH)(CO)(CH₂)ₙ(CO)(NH), wherein n is is 0 to 10.

10. A nucleic acid according to Claim 9 wherein the nucleic acid is 25-50 nucleotides in length; and R₁ is selected from the group consisting of a bond between the nitrogens, (NH)(CO)(NH) and (NH)(CO)(CH₂)₄(CO)(NH).

11. A method of making a nucleic acid probe which comprises a single-stranded nucleic acid and a catalytically active enzyme, wherein said nucleic acid comprises a segment of at least 20 nucleotides in the sequence complementary to the sequence of the target segment of said probe; wherein said nucleic acid and said enzyme are linked by a linker of formula I:
-(PO₃)NH(R₁)N=CH(R₂)(CO)- I
wherein R₁ is selected from the group consisting of a bond between the nitrogens, (NH)(CO)(NH), 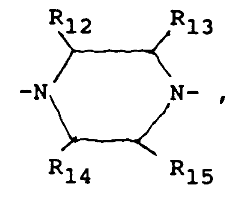 and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein R₂ is selected from the group consisting of alkylene of 1 to 20 carbon atoms or arylene of formula: 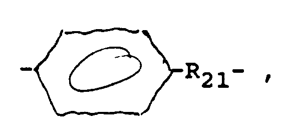 wherein R₂₁ is alkylene of 0 to 10 carbon atoms; wherein the -(PO₃)- group is bonded to the 5'-carbon of the 5'-nucleotide of the nucleic acid; and wherein the carbon of the carbonyl group at the right-hand side of figure I is bonded to the nitrogen of a free amino group of the enzyme, which method comprises reacting, in an aqueous solution buffered at a pH between 7.0 and 9.0, a single-stranded nucleic acid, which has the same sequence as that of the nucleic acid of the probe and is derivatized with a moiety of a formula XIV
-(PO₃)(NH)(R₁)NH₂ XIV
wherein the -(PO₃)- group is bonded to the 5'-carbon of the 5'-nucleotide of the nucleic acid, with a catalytically active enzyme, which is derivatized at a free amino group with a moiety of formula XVIII
(CHO)(R₂)(CO)- XVIII.

12. A method according to Claim 11 wherein the nucleic acid consists of 20-150 nucleotides in the sequence complementary to the sequence of the target segment of the probe; the catalytically active enzyme is selected from the group consisting of phosphatases, peroxidases, beta-galactosidases, ureases, carbonic anhydrases and luciferases; R₁ is selected from the group consisting of a bond between the nitrogens, (NH)(CO)(NH) and (NH)(CO)(CH₂)ₙ(CO)(NH), wherein n is is 0 to 10; and R₂ is selected from the group consisting of p-phenylene and (CH₂)ₘ, wherein m is 1 to 10.

13. A method according to Claim 12 wherein the nucleic acid is 25-50 nucleotides in length; R₁ is selected from the group consisting of a bond between the nitrogens, (NH)(CO)(NH) and (NH)(CO)(CH₂)₄(CO)(NH); and R₂ is p-phenylene.

14. A method according to any one of Claims 11 to 13 wherein the enzyme is selected from the group consisting of calf intestine alkaline phosphatase, E. coli alkaline phosphatase, horseradish peroxidase, E. coli beta-galactosidase, jack bean urease, bovine erythrocyte carbonic anhydrase, P. fischeri luciferase and firefly luciferase.

15. A method of making a nucleic acid probe which comprises a single-stranded nucleic acid and a catalytically active enzyme, wherein said nucleic acid comprises a segment of at least 20 nucleotides in the sequence complementary to the sequence of the target segment of said probe; wherein said enzyme is a glycoprotein that has been oxidized by treatment with periodate; wherein said nucleic acid and said enzyme are linked by a linker of a formula IX
-(PO₃)NH(R₁)N= IX
wherein R₁ is selected from the group consisting of a bond between NH and N, (NH)(CO)(NH), 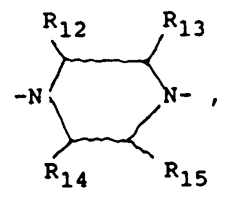 and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein the -(PO₃)- group is bonded to the 5'-carbon of the 5'-nucleotide of the nucleic acid; and wherein the -N= at the right-hand side of formula IX is bonded to a carbon of a sugar residue of the enzyme, that was the carbon of a carbonyl group formed in the treatment of the enzyme with periodate, which method comprises reacting, in an aqueous solution buffered at a pH between 7.0 and 9.0, a single-stranded nucleic acid, which has the same sequence as that of the nucleic acid probe and is derivatized with a moiety of a formula XIV
-(PO₃)(NH)(R₁)NH₂ XIV
wherein the -(PO₃)- group is bonded to the 5'-carbon of the 5'-nucleotide, with said catalytically active enzyme.

16. A method according to Claim 15 wherein the nucleic acid consists of 20-150 nucleotides in the sequence complementary to the sequence of the target segment of the probe; the catalytically active enzyme is an eukaryotic peroxidase, e.g. horseradish peroxidase; and R₁ is selected from the group consisting of a bond between the nitrogens, (NH)(CO)(NH) and (NH)(CO)(CH₂)ₙ(CO)(NH), wherein n is 0 to 10.

17. A method according to Claim 16 wherein the nucleic acid is 25-50 nucleotides in length; and R₁ is selected from the group consisting of a bond between the nitrogens, (NH)(CO)(NH) and (NH)(CO)(CH₂)₄(CO)(NH).

18. A method of making a first nucleic acid probe which comprises a single-stranded nucleic acid and a catalytically active enzyme, wherein said nucleic acid comprises a segment of at least 20 nucleotides in the sequence complementary to the sequence of the target segment of said probe; wherein said nucleic acid and said enzyme are linked by a first linker of formula II:
-(PO₃)NH(R₁)NH(CH₂)(R₂)(CO)- II
or formula XXIX
-(PO₃)NH(R₁)NH- XXIX
wherein R₁ is selected from the group consisting of a bond between the nitrogens, (NH)(CO)(NH), 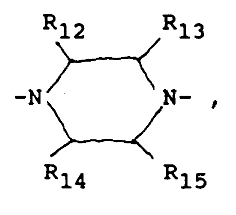 and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein R₂ is selected from the group consisting of alkylene of 1 to 20 carbon atoms and arylene of formula: 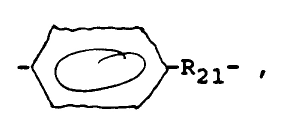 wherein R₂₁ is alkylene of 0 to 10 carbon atoms; and wherein the -(PO₃)- group is bonded to the 5'-carbon of the 5'-nucleotide of the nucleic acid; provided that, if the formula of said first linker is formula II, the carbon of the -(CO)- group at the right-hand side of said formula is bonded to a free amino group of the enzyme; and provided further that, if the formula of said first linker is formula XXIX, the catalytically active enzyme is a glycoprotein and has been oxidized with periodate and the nitrogen of the -NH- group at the right-hand side of said formula is bonded to a carbon of a sugar residue of the enzyme, that was the carbon of a carbonyl group formed in the treatment of the enzyme with periodate; which method comprises reacting, in an aqueous solution buffered at a pH of 5 to 9, an alkali metal salt of cyanoborohydride with a second nucleic acid probe which is substantially the same as said first nucleic acid probe except that, in said second probe, the nucleic acid and enzyme are linked by a second linker of formula I
-(PO₃)NH(R₁)N=CH(R₂)(CO)- I
or formula IX
-(PO₃)NH(R₁)N= IX
in place of said first linker, provided that, if said first linker is of formula II, said second linker is of formula I, and, if said first linker is of formula XXIX, said second linker is of formula IX.

19. A method according to Claim 18 wherein the first linker is of formula II, wherein the nucleic acid consists of 20-150 nucleotides in the sequence complementary to the sequence of the target segment; the catalytically active enzyme is selected from the group consisting of phosphatases, peroxidases, beta-galactosidases, ureases, carbonic anhydrases and luciferases; and R₁ is selected from the group consisting of a bond between the nitrogens, (NH)(CO)(NH) and (NH)(CO)(CH₂)ₙ(CO)(NH), wherein n is is 0 to 10; and R₂ is selected from the group consisting of p-phenylene and (CH₂)ₘ, wherein m is 1 to 10.

20. A method according to Claim 19 wherein the nucleic acid is 25-50 nucleotides in length; R₁ is selected from the group consisting of a bond between the nitrogens, (NH)(CO)(NH) and (NH)(CO)(CH₂)₄(CO)(NH); and R₂ is p-phenylene.

21. A method according to any one of Claims 18 to 20 wherein the enzyme is selected from the group consisting of calf intestine alkaline phosphatase, E. coli alkaline phosphatase, horseradish peroxidase, E. coli betagalactosidase, jack bean urease, bovine erythrocyte carbonic anhydrase, P. fischeri luciferase and firefly luciferase.

22. A method according to Claim 18 wherein the first linker is of formula XXIX; the nucleic acid consists of 20-150 nucleotides in the sequence complementary to the sequence of the target sequence; the catalytically active enzyme is an eukaryotic peroxidase, e.g. horseradish peroxidase; and R₁ is selected from the group consisting of a bond between the nitrogens, (NH)(CO)(NH) and (NH)(CO)(CH₂)ₙ(CO)(NH), wherein n is 0 to 10.

23. A method according to Claim 22 wherein the nucleic acid is 25-50 nucleotides in length; and R₁ is selected from the group consisting of a bond between the nitrogens, (NH)(CO)(NH) and (NH)(CO)(CH₂)₄ (CO)(NH).

24. A nucleic acid probe hybridization assay for a nucleic acid analyte, which comprises a target segment of at least 20 nucleotides in a known sequence, which assay comprises employing as the nucleic acid probe for detecting nucleic acid analyte in said assay a probe which comprises a single-stranded nucleic acid and a catalytically active enzyme, wherein the nucleic acid of the probe comprises a segment with the sequence complementary to the sequence of said target segment; wherein said nucleic acid and said enzyme are linked by a linker of a formula selected from the group consisting of formula I
-(PO₃)NH(R₁)N=CH(R₂)(CO)- I
formula II
-(PO₃)NH(R₁)NH(CH₂)(R₂)(CO)- II
formula IX
-(PO₃)NH(R₁)N= IX
and formula X
-(PO₃)NH(R₁)NH- X
wherein R₁ is selected from the group consisting of a bond between the nitrogens, (NH)(CO)(NH), 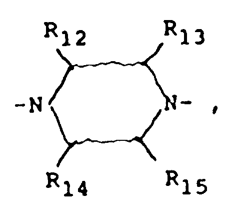 and (NH)(CO)(R₁₁)(CO)(NH), wherein R₁₁ is a bond between the carbons of the (CO) groups or alkylene of 1 to 10 carbon atoms and wherein R₁₂ R₁₃, R₁₄ and R₁₅ are the same or different and each is selected from the group consisting of hydrogen and alkyl of 1 to 3 carbon atoms; wherein R₂ is selected from the group consisting of alkylene of 1 to 20 carbon atoms and arylene of formula: 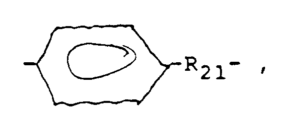 wherein R₂₁ is alkylene of 0 to 10 carbon atoms; wherein the -(PO₃)- group is bonded to the 5'-carbon of the 5'-nucleotide of the nucleic acid; and wherein the carbons of the carbonyl groups at the right-hand sides of figures I and II are bonded to the nitrogen of a free amino group of the enzyme; provided that, if the linker is of formula IX or formula X, the catalytically active enzyme is a glycoprotein that has been oxidized with periodate and -N= at the right-hand side of formula IX and the nitrogen of the -NH- group at the right-hand side of formula X are bonded to a carbon of a sugar residue of the enzyme, that was the carbon of a carbonyl group formed in the treatment of the enzyme with periodate.

25. An assay according to Claim 24 wherein the linker of the probe is of formula I or formula II, the nucleic acid of the probe consists of 20-150 nucleotides in the sequence complementary to the sequence of the target segment; the catalytically active enzyme is selected from the group consisting of phosphatases, peroxidases, beta-galactosidases, ureases, carbonic anhydrases and luciferases; R₁ is selected from the group consisting of a bond between the nitrogens, (NH)(CO)(NH) and (NH)(CO)(CH₂)ₙ(CO)(NH), wherein n is is 0 to 10; and R₂ is selected from the group consisting of p-phenylene and (CH₂)ₘ, wherein m is 1 to 10.

26. An assay according to Claim 25 wherein the nucleic acid of the probe is 25-50 nucleotides in length; R₁ is selected from the group consisting of a bond between the nitrogens, (NH)(CO)(NH) and (NH)(CO)(CH₂)₄(CO)(NH); and R₂ is p-phenylene.

27. An assay according to any one of Claims 24 to 26 wherein the enzyme of the probe is selected from the group consisting of calf intestine alkaline phosphatase, E. coli alkaline phosphatase, horseradish peroxidase, E. coli beta-galactosidase, jack bean urease, bovine erythrocyte carbonic anhydrase, P. fishcheri luciferase and firefly luciferase.

28. An assay according to any one of Claims 25 to 27 wherein the linker of the probe is of formula I.

29. An assay according to Claim 24 wherein the linker of the probe is of formula IX or formula X, the nucleic acid of the probe consists of 20-150 nucleotides in the sequence complementary to the sequence of the target segment; the catalytically active enzyme is an eukaryotic peroxidase, e.g. horseradish peroxidase; and R₁ is selected from the group consisting of a bond between the nitrogens, (NH)(CO)(NH) and (NH)(CO)(CH₂)ₙ(CO)(NH), wherein n is 0 to 10.

30. An assay according to Claim 29 wherein the nucleic acid of the proe is 25-50 nucleotides in length; and R₁ is selected from the group consisting of a bond between the nitrogens, (NH)(CO)(NH) and (NH)(CO)(CH₂)₄(CO)(NH).

31. An assay according to Claim 29 or Claim 30 wherein the linker of the probe is of formula IX.

32. A probe according to any one of Claims 1 to 7, or a nucleic acid according to any one of Claims 8 to 10, or a method according to any one of Claims 11 to 23, or an assay according to any one of Claims 24 to 31, wherein the nucleic acid, or in the case of the said assay the nucleic acid of the probe, is a DNA.

33. The use of a probe according to any one of Claims 1 to 7 or of a nucleic acid according to any one of Claims 8 to 10 or 32 in a hybridization assay or in making a probe, respectively.

## Patentansprüche

1. Nukleinsäuresonde, welche eine einzelsträngige Nukleinsäure und ein katalytisch aktives Enzym umfaßt, worin die Nukleinsäure ein Segment von mindestens 20 Nukleotiden in der komplementären Sequenz zu der Sequenz des Zielsegments der Sonde umfaßt; worin die Nukleinsäure und das Enzym durch einen Linker einer Formel verknüpft sind, welche gewählt wird aus der aus Formel I
-(PO₃)NH(R₁)N=CH(R₂)(CO)- I
und Formel II
-(PO₃)NH(R₁)NH(CH₂)(R₂)(CO)- II
bestehenden Gruppe, worin R₁ aus der Gruppe gewählt wird, die aus einer Bindung zwischen den Stickstoffatomen, (NH)(CO)(NH), 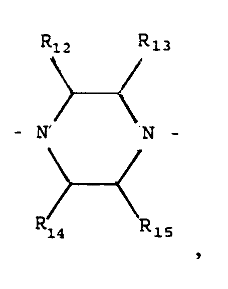 und (NH)(CO)(R₁₁)(CO)(NH) besteht, worin R₁₁ eine Bindung zwischen den Kohlenstoffatomen der (CO)-Gruppen oder ein Alkylen mit 1 bis 10 Kohlenstoffatomen ist, und worin R₁₂, R₁₃, R₁₄ und R₁₅ gleich oder verschieden sind und jeweils aus der aus Wasserstoff und Alkyl mit 1 bis 3 Kohlenstoffatomen bestehenden Gruppe gewählt sind; worin R₂ gewählt wird aus der Gruppe, welche aus Alkylen mit 1 bis 20 Kohlenstoffatomen und Arylen der Formel: 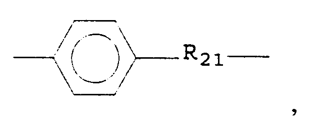 worin R₂₁ Alkylen von 0 bis 10 Kohlenstoffatomen ist, besteht; worin die -(PO₃)-Gruppe an das 5'-Kohlenstoffatom des 5'-Nukleotids der Nukleinsäure gebunden ist; und worin die Kohlenstoffatome der Carbonylgruppen an den rechts gelegenen Seiten der Figuren I und II an das Stickstoffatom einer freien Aminogruppe des Enzyms gebunden sind.

2. Nukleinsäuresonde nach Anspruch 1, worin die Nukleinsäure aus 20-150 Nukleotiden in der komplementären Sequenz zu der Sequenz des Zielsegments besteht; das katalytisch aktive Enzym aus der aus Phosphatasen, Peroxidasen, β-Galactosidasen, Ureasen, Carboanhydrasen und Luciferasen bestehenden Gruppe gewählt wird; R₁ aus der aus einer Bindung zwischen den Stickstoffatomen, (NH)(CO)(NH) und (NH)(CO)(CH₂)ₙ(CO)(NH) bestehenden Gruppe gewählt wird, worin n 0 bis 10 ist; und R₂ aus der aus p-Phenylen und (CH₂)ₘ bestehenden Gruppe gewählt wird, worin m 1 bis 10 ist.

3. Nukleinsäuresonde nach Anspruch 2, worin die Nukleinsäure eine Länge von 25-50 Nukleotiden aufweist; R₁ aus der aus einer Bindung zwischen den Stickstoffatomen, (NH)(CO)(NH) und (NH)(CO)(CH₂)₄(CO)(NH) bestehenden Gruppe gewählt wird; und R₂ p-Phenylen ist.

4. Nukleinsäuresonde nach einem der Ansprüche 1 bis 3, worin das Enzym aus der aus Alkalischer Phosphatase aus Kälberdarm, E.coli-Alkalischer Phosphatase, Meerrettichperoxidase, E.coli-β-Galactosidase, Schwertbohnen-Urease, Rindererythrocyten-Carboan-hydrase, P.fischeri-Luciferase und Glühwürmchen-Luciterase bestehenden Gruppe gewählt wird.

5. Nukleinsäuresonde, welche eine einzelsträngige Nukleinsäure und ein katalytisch aktives Enzym umfaßt, worin die Nukleinsäure ein Segment von mindestens 20 Nukleotiden in der komplementären Sequenz zu der Sequenz des Zielsegments der Sonde umfaßt; worin das Enzym ein Glykoprotein ist, das mittels Behandlung mit Periodat oxidiert worden ist; worin die Nukleinsäure und das Enzym durch einen Linker einer Formel verknüpft sind, welche gewählt wird aus der aus Formel IX
-(PO₃)NH(R₁)N= IX
und Formel X
-(PO₃)NH(R₁)NH- X
bestehenden Gruppe, worin R₁ aus der Gruppe gewählt wird, die aus einer Bindung zwischen den Stickstoffatomen, (NH)(CO)(NH), 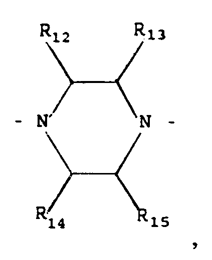 und (NH)(CO)(R₁₁)(CO)(NH) besteht, worin R₁₁ eine Bindung zwischen den Kohlenstoffatomen der (CO)-Gruppen oder ein Alkylen mit 1 bis 10 Kohlenstoffatomen ist, und worin R₁₂, R₁₃, R₁₄ und R₁₅ gleich oder verschieden sind und jeweils aus der aus Wasserstoff und Alkyl mit 1 bis 3 Kohlenstoffatomen bestehenden Gruppe gewählt sind; worin die -(PO₃)-Gruppe an das 5'-Kohlenstoffatom des 5'-Nukleotids der Nukleinsäure gebunden ist; und worin das -N= an der rechts gelegenen Seite der Formel IX und der Stickstoff der -NH-Gruppe an der rechts gelegenen Seite der Formel X an ein Kohlenstoffatom eines Zuckerrestes des Enzyms gebunden sind, das das Kohlenstoffatom einer bei der Behandlung des Enzyms mit Periodat gebildeten Carbonylgruppe war.

6. Nukleinsäuresonde nach Anspruch 5, worin die Nukleinsäure aus 20-150 Nukleotiden in der komplementären Sequenz zu der Sequenz des Zielsegments besteht; das katalytisch aktive Enzym eine eukaryotische Peroxidase ist, z.B. Meerrettichperoxidase; und R₁ aus der aus einer Bindung zwischen den Stickstoffatomen, (NH)(CO)(NH) und (NH)(CO)(CH₂)ₙ(CO)(NH) bestehenden Gruppe gewählt wird, worin n 0 bis 10 ist.

7. Nukleinsäuresonde nach Anspruch 6, worin die Nukleinsäure eine Länge von 25-50 Nukleotiden aufweist; und R₁ aus der aus einer Bindung zwischen den Stickstoffatomen, (NH)(CO)(NH) und (NH)(CO)(CH₂)₄(CO)(NH) bestehenden Gruppe gewählt wird.

8. Einzelsträngige Nukleinsäure, welche ein Intermediat bei der Herstellung einer Nukleinsäuresonde zum Nachweis eines Zielsegments ist, ein Segment von mindestens 20 Nukleotiden in der komplementären Sequenz zu der des Zielsegments umfaßt und mit einem Rest der Formel XIV
-(PO₃)NH(R₁)NH₂ XIV
derivatisiert ist, worin R₁ aus der Gruppe gewählt wird, die aus einer Bindung zwischen den Stickstoffatomen, (NH)(CO)(NH), 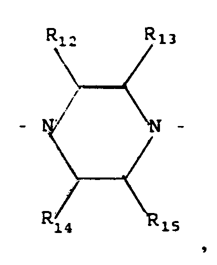 und (NH)(CO)(R₁₁)(CO)(NH) besteht, worin R₁₁ eine Bindung zwischen den Kohlenstoffatomen der (CO)-Gruppen oder ein Alkylen mit 1 bis 10 Kohlenstoffatomen ist, und worin R₁₂, R₁₃, R₁₄ und R₁₅ gleich oder verschieden sind und jeweils aus der aus Wasserstoff und Alkyl mit 1 bis 3 Kohlenstoffatomen bestehenden Gruppe gewählt sind; und worin die -(PO₃)-Gruppe an das 5'-Kohlenstoffatom des 5'-Nukleotids der Nukleinsäure gebunden ist.

9. Nukleinsäure nach Anspruch 8, worin die Nukleinsäure aus 20-150 Nukleotiden in der komplementären Sequenz zu der Sequenz des Zielsegments besteht; und R₁ aus der aus einer Bindung zwischen den Stickstoffatomen, (NH)(CO)(NH) und (NH)(CO)(CH₂)ₙ(CO)(NH) bestehenden Gruppe gewählt wird, worin n 0 bis 10 ist.

10. Nukleinsäure nach Anspruch 9, worin die Nukleinsäure eine Länge von 25-50 Nukleotiden aufweist; und R₁ aus der aus einer Bindung zwischen den Stickstoffatomen, (NH)(CO)(NH) und (NH)(CO)(CH₂)₄(CO)(NH) bestehenden Gruppe gewählt wird.

11. Verfahren zur Herstellung einer Nukleinsäuresonde, welche eine einzelsträngige Nukleinsäure und ein katalytisch aktives Enzym umfaßt, worin die Nukleinsäure ein Segment von mindestens 20 Nukleotiden in der komplementären Sequenz zu der Sequenz des Zielsegments der Sonde umfaßt; worin die Nukleinsäure und das Enzym durch einen Linker der Formel I:
-(PO₃)NH(R₁)N=CH(R₂)(CO)- I
verknüpft sind, worin R₁ aus der Gruppe gewählt wird, die aus einer Bindung zwischen da Stickstoffatomen, (NH)(CO)(NH), 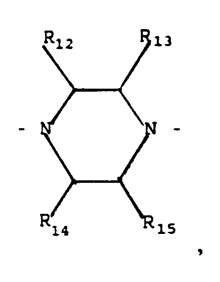 und (NH)(CO)(R₁₁)(CO)(NH) besteht, worin R₁₁ eine Bindung zwischen den Kohlenstoffatomen der (CO)-Gruppen oder ein Alkylen mit 1 bis 10 Kohlenstoffatomen ist, und worin R₁₂, R₁₃, R₁₄ und R₁₅ gleich oder verschieden sind und jeweils aus der aus Wasserstoff und Alkyl mit 1 bis 3 Kohlenstoffatomen bestehenden Gruppe gewählt sind; worin R₂ gewählt wird aus der Gruppe, welche aus Alkylen mit 1 bis 20 Kohlenstoffatomen und Arylen der Formel: 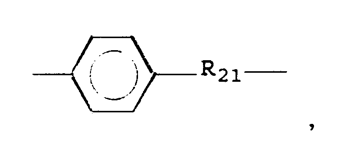 worin R₂₁ Alkylen mit 0 bis 10 Kohlenstoffatomen ist, besteht; worin die -(PO₃)-Gruppe an das 5'-Kohlenstoffatom des 5'-Nukleotids der Nukleinsäure gebunden ist; und worin das Kohlenstoffatom der Carbonylgruppe an der rechts gelegenen Seite der Figur I an das Stickstoffatom einer freien Aminogruppe des Enzyms gebunden ist, wobei das Verfahren das Umsetzen einer einzelsträngigen Nukleinsäure, welche dieselbe Sequenz wie die der Nukleinsäure der Sonde besitzt und mit einem Rest der Formel XIV
-(PO₃)NH(R₁)NH₂ XIV
derivatisiert ist, worin die -(PO₃)-Gruppe an das 5'-Kohlenstoffatom des 5'-Nukleotids der Nukleinsäure gebunden ist, mit einem katalytisch aktiven Enzym, welches an der freien Aminogruppe mit einem Rest der Formel XVIII
(CHO)(R₂)(CO)- XVIII
derivatisiert ist, in einer wäßrigen Lösung, gepuffert auf einen pH-Wert zwischen 7,0 und 9,0, umfaßt.

12. Verfahren nach Anspruch 11, worin die Nukleinsäure aus 20-150 Nukleotiden in der komplementären Sequenz zu der Sequenz des Zielsegments der Sonde besteht; das katalytisch aktive Enzym aus der aus Phosphatasen, Peroxidasen, β-Galactosidasen, Ureasen, Carboanhydrasen und Luciferasen bestehenden Gruppe gewählt wird; R₁ aus der aus einer Bindung zwischen den Stickstoffatomen, (NH)(CO)(NH) und (NH)(CO)(CH₂)ₙ(CO)(NH) bestehenden Gruppe gewählt wird, worin n 0 bis 10 ist; und R₂ aus der aus p-Phenylen und (CH₂)ₘ bestehenden Gruppe gewählt wird, worin m 1 bis 10 ist.

13. Verfahren nach Anspruch 12, worin die Nukleinsäure eine Länge von 25-50 Nukleotiden aufweist; R₁ aus der aus einer Bindung zwischen den Stickstoffatomen, (NH)(CO)(NH) und (NH)(CO)(CH₂)₄(CO)(NH) bestehenden Gruppe gewählt wird; und R₂ p-Phenylen ist.

14. Verfahren nach einem der Ansprüche 11 bis 13, worin das Enzym aus der aus Alkalischer Phosphatase aus Kälberdarm, E.coli-Alkalischer Phosphatase, Meerrettichperoxidase, E.coli-β-Galactosidase, Schwertbohnen-Urease, Rindererythrocyten-Carboanhydrase, P.fischeri-Luciferase und Glühwürmchen-Luciferase bestehenden Gruppe gewählt wird.

15. Verfahren zur Herstellung einer Nukleinsäuresonde, welche eine einzelsträngige Nukleinsäure und ein katalytisch aktives Enzym umfaßt, worin die Nukleinsäure ein Segment von mindestens 20 Nukleotiden in der komplementären Sequenz zu der Sequenz des Zielsegments der Sonde umfaßt; worin das Enzym ein Glykoprotein ist, das mittels Behandlung mit Periodat oxidiert worden ist; worin die Nukleinsäure und das Enzym durch einen Linker der Formel IX
-(PO₃)NH(R₁)N= IX
verknüpft sind, worin R₁ aus der Gruppe gewählt wird, die aus einer Bindung zwischen NH und N, (NH)(CO)(NH), 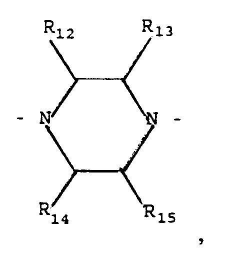 und (NH)(CO)(R₁₁)(CO)(NH) besteht, worin R₁₁ eine Bindung zwischen den Kohlenstoffatomen der (CO)-Gruppen oder ein Alkylen mit 1 bis 10 Kohlenstoffatomen ist, und worin R₁₂, R₁₃, R₁₄ und R₁₅ gleich oder verschieden sind und jeweils aus der aus Wasserstoff und Alkyl mit 1 bis 3 Kohlenstoffatomen bestehenden Gruppe gewählt sind; worin die -(PO₃)-Gruppe an das 5'-Kohlenstoffatom des 5'-Nukleotids der Nukleinsäure gebunden ist; und worin das -N= an der rechts gelegenen Seite der Formel IX an ein Kohlenstoffatom eines Zuckerrestes des Enzyms gebunden ist, das das Kohlenstoffatom einer bei der Behandlung des Enzyms mit Periodat gebildeten Carbonylgruppe war, wobei das Verfahren das Umsetzen einer einzelsträngigen Nukleinsäure, welche dieselbe Sequenz wie die der Nukleinsäure der Sonde besitzt und mit einem Rest der Formel XIV
-(PO₃)NH(R₁)NH₂ XIV
derivatisiert ist, worin die -(PO₃)-Gruppe an das 5'-Kohlenstoffatom des 5'-Nukleotids gebunden ist, mit dem katalytisch aktiven Enzym in einer wäßrigen Lösung, gepuffert auf einen pH-Wert zwischen 7,0 und 9,0, umfaßt.

16. Verfahren nach Anspruch 15, worin die Nukleinsäure aus 20-150 Nukleotiden in der komplementären Sequenz zu der Sequenz des Zielsegments der Sonde besteht; das katalytisch aktive Enzym eine eukaryotische Peroxidase, z.B. Meerrettichperoxidase, ist; und R₁ aus der aus einer Bindung zwischen den Stickstoffatomen, (NH)(CO)(NH) und (NH)(CO)(CH₂)ₙ(CO)(NH) bestehenden Gruppe gewählt wird, worin n 0 bis 10 ist.

17. Verfahren nach Anspruch 16, worin die Nukleinsäure eine Länge von 25-50 Nukleotiden aufweist; und R₁ aus der aus einer Bindung zwischen den Stickstoffatomen, (NH)(CO)(NH) und (NH)(CO)(CH₂)₄(CO)(NH) bestehenden Gruppe gewählt wird.

18. Verfahren zur Herstellung einer ersten Nukleinsäuresonde, welche eine einzelsträngige Nukleinsäure und ein katalytisch aktives Enzym umfaßt, worin die Nukleinsäure ein Segment von mindestens 20 Nukleotiden in der komplementären Sequenz zu der Sequenz des Zielsegments der Sonde umfaßt; worin die Nukleinsäure und das Enzym verknüpft sind durch einen ersten Linker der Formel II:
-(PO₃)NH(R₁)NH(CH₂)(R₂)(CO)- II
oder der Formel XXIX
-(PO₃)NH(R₁)NH- XXIX,
worin R₁ aus der Gruppe gewählt wird, die aus einer Bindung zwischen den Stickstoffatomen, (NH)(CO)(NH), 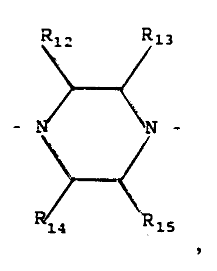 und (NH)(CO)(R₁₁)(CO)(NH) besteht, worin R₁₁ eine Bindung zwischen den Kohlenstoffatomen der (CO)-Gruppen oder ein Alkylen mit 1 bis 10 Kohlenstoffatomen ist, und worin R₁₂, R₁₃, R₁₄ und R₁₅ gleich oder verschieden sind und jeweils aus der aus Wasserstoff und Alkyl mit 1 bis 3 Kohlenstoffatomen bestehenden Gruppe gewählt sind; worin R₂ gewählt wird aus der Gruppe, welche aus Alkylen mit 1 bis 20 Kohlenstoffatomen und Arylen der Formel: 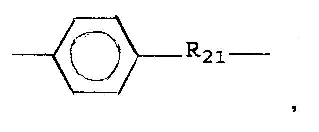 worin R₂₁ Alkylen mit 0 bis 10 Kohlenstoffatomen ist, besteht; und worin die -(PO₃)-Gruppe an das 5'-Kohlenstoffatom des 5'-Nukleotids der Nukleinsäure gebunden ist; mit der Maßgabe, daß, wenn die Formel des ersten Linkers die Formel II ist, das Kohlenstoffatom der -(CO)-Gruppe an der rechts gelegenen Seite der Formel an eine freie Aminogruppe des Enzyms gebunden ist; und ferner mit der Maßgabe, daß, wenn die Formel des ersten Linkers die Formel XXIX ist, das katalytisch aktive Enzym ein Glykoprotein ist und mit Periodat oxidiert worden ist, und das Stickstoffatom der -NH-Gruppe an der rechts gelegenen Seite der Formel an ein Kohlenstoffatom eines Zuckerrestes des Enzyms gebunden ist, das das Kohlenstoffatom einer bei der Behandlung des Enzyms mit Periodat gebildeten Carbonylgruppe war; wobei das Verfahren das Umsetzen eines Alkalimetallsalzes von Cyanoborhydrid mit einer zweiten Nukleinsäuresonde in einer wäßrigen Lösung, gepuffert auf einen pH-Wert zwischen 7,0 und 9,0, umfaßt, welche im wesentlichen die gleiche ist wie die erste Nukleinsäuresonde mit der Ausnahme, daß in der zweiten Sonde die Nukleinsäure und das Enzym durch einen zweiten Linker der Formel I
-(PO₃)NH(R₁)N=CH(R₂)(CO)- I
oder der Formel IX
-(PO₃)NH(R₁)N= IX
anstelle des ersten Linkers verknüpft sind, mit der Maßgabe, daß, wenn der erste Linker von der Formel II ist, der zweite Linker von der Formel I ist, und daß, wenn der erste Linker von der Formel XXIX ist, der zweite Linker von der Formel IX ist.

19. Verfahren nach Anspruch 18, worin der erste Linker von der Formel II ist, worin die Nukleinsäure aus 20-150 Nukleotiden in der komplementären Sequenz zu der Sequenz des Zielsegments besteht; das katalytisch aktive Enzym aus der aus Phosphatasen, Peroxidasen, β-Galactosidasen, Ureasen, Carboanhydrasen und Luciferasen bestehenden Gruppe gewählt wird; und R₁ aus der aus einer Bindung zwischen den Stickstoffatomen, (NH)(CO)(NH) und (NH)(CO)(CH₂)ₙ(CO)(NH) bestehenden Gruppe gewählt wird, worin n 0 bis 10 ist; und R₂ aus der aus p-Phenylen und (CH₂)ₘ bestehenden Gruppe gewählt wird, worin m 1 bis 10 ist.

20. Verfahren nach Anspruch 19, worin die Nukleinsäure eine Länge von 25-50 Nukleotiden aufweist; R₁ aus der aus einer Bindung zwischen den Stickstoffatomen, (NH)(CO)(NH) und (NH)(CO)(CH₂)₄(CO)(NH) bestehenden Gruppe gewählt wird; und R₂ p-Phenylen ist.

21. Verfahren nach einem der Ansprüche 18 bis 20, worin das Enzym aus der aus Alkalischer Phosphatase aus Kälberdarm, E.coli-Alkalischer Phosphatase, Meerrettichperoxidase, E.coli-β-Galactosidase, Schwertbohnen-Urease, Rindererythrocyten-Carboanhydrase, P.fischeri-Luciferase und Glühwürmchen-Luciferase bestehenden Gruppe gewählt wird.

22. Verfahren nach Anspruch 18, worin der erste Linker von der Formel XXIX ist; die Nukleinsäure aus 20-150 Nukleotiden in der komplementären Sequenz zu der Sequenz der Zielsequenz besteht; das katalytisch aktive Enzym eine eukaryotische Peroxidase, z.B. Meerrettichperoxidase, ist; und R₁ aus der aus einer Bindung zwischen den Stickstoffatomen, (NH)(CO)(NH) und (NH)(CO)(CH₂)ₙ(CO)(NH) bestehenden Gruppe gewählt wird, worin n 0 bis 10 ist.

23. Verfahren nach Anspruch 22, worin die Nukleinsäure eine Länge von 25-50 Nukleotiden aufweist; und R₁ aus der aus einer Bindung zwischen den Stickstoffatomen, (NH)(CO)(NH) und (NH)(CO)(CH₂)₄(CO)(NH) bestehenden Gruppe gewählt wird.

24. Nukleinsäuresonden-Hybridisierungsassay für einen Nukleinsäureanalyten, der ein Zielsegment mit mindestens 20 Nukleotiden in einer bekannten Sequenz umfaßt, wobei der Assay das Anwenden einer Sonde als Nukleinsäuresonde zum Nachweis des Nukleinsäureanalyten in dem Assay umfaßt, welche eine einzelsträngige Nukleinsäure und ein katalytisch aktives Enzym umfaßt, worin die Nukleinsäure der Sonde ein Segment mit der komplementären Sequenz zu der Sequenz der Zielsegments umfaßt; worin die Nukleinsäure und das Enzym durch einen Linker einer Formel verknüpft sind, welche gewählt wird aus der Gruppe, bestehend aus Formel I
-(PO₃)NH(R₁)N=CH(R₂)(CO)- I
Formel II
-(PO₃)NH(R₁)NH(CH₂)(R₂)(CO)- II
Formel IX
-(PO₃)NH(R₁)N= IX
und Formel X
-(PO₃)NH(R₁)NH- X
worin R₁ aus der Gruppe gewählt wird, die aus einer Bindung zwischen den Stickstoffatomen, (NH)(CO)(NH), 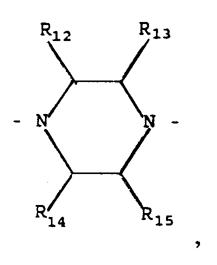 und (NH)(CO)(R₁₁)(CO)(NH) besteht, worin R₁₁ eine Bindung zwischen den Kohlenstoffatomen der (CO)-Gruppen oder ein Alkylen mit 1 bis 10 Kohlenstoffatomen ist, und worin R₁₂, R₁₃, R₁₄ und R₁₅ gleich oder verschieden sind und jeweils aus der aus Wasserstoff und Alkyl mit 1 bis 3 Kohlenstoffatomen bestehenden Gruppe gewählt sind; worin R₂ gewählt wird aus der Gruppe, welche aus Alkylen mit 1 bis 20 Kohlenstoffatomen und Arylen der Formel: 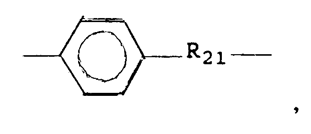 worin R₂₁ Alkylen mit 0 bis 10 Kohlenstoffatomen ist, besteht; worin die -(PO₃)-Gruppe an das 5'-Kohlenstoffatom des 5'-Nukleotids der Nukleinsäure gebunden ist; und worin die Kohlenstoffatome der Carbonylgruppen an den rechts gelegenen Seiten der Figuren I und II an das Stickstoffatom einer freien Aminogruppe des Enzyms gebunden sind; mit der Maßgabe, daß, wenn der Linker von der Formel IX oder Formel X ist, das katalytisch aktive Enzym ein Glykoprotein ist, das mit Periodat oxidiert worden ist, und -N= an der rechts gelegenen Seite der Formel IX und das Stickstoffatom der -NH-Gruppe an der rechts gelegenen Seite der Formel X an ein Kohlenstoffatom eines Zuckerrestes des Enzyms gebunden sind, das das Kohlenstoffatom einer bei der Behandlung des Enzyms mit Periodat gebildeten Carbonylgruppe war.

25. Assay nach Anspruch 24, worin der Linker der Sonde von der Formel I oder der Formel II ist, worin die Nukleinsäure der Sonde aus 20-150 Nukleotiden in der komplementären Sequenz zu der Sequenz des Zielsegments besteht; das katalytisch aktive Enzym aus der aus Phosphatasen, Peroxidasen, β-Galactosidasen, Ureasen, Carboanhydrasen und Luciferasen bestehenden Gruppe gewählt wird; R₁ aus der aus einer Bindung zwischen den Stickstoffatomen, (NH)(CO)(NH) und (NH)(CO)(CH₂)ₙ(CO)(NH) bestehenden Gruppe gewählt wird, worin n 0 bis 10 ist; und R₂ aus der aus p-Phenylen und (CH₂)ₘ bestehenden Gruppe gewählt wird, worin m 1 bis 10 ist.

26. Assay nach Anspruch 25, worin die Nukleinsäure der Sonde eine Länge von 25-50 Nukleotiden aufweist; R₁ aus der aus einer Bindung zwischen den Stickstoffatomen, (NH)(CO)(NH) und (NH)(CO)(CH₂)₄(CO)(NH) bestehenden Gruppe gewählt wird; und R₂ p-Phenylen ist.

27. Assay nach einem der Ansprüche 24 bis 26, worin das Enzym der Sonde aus der aus Alkalischer Phosphatase aus Kälberdarm, E.coli-Alkalischer Phosphatase, Meerrettichperoxidase, E.coli-β-Galactostdase, Schwertbohnen-Urease, Rindererythrocyten-Carboanhydrase, P.fischeri-Luciferase und Glühwürmchen-Luciferase bestehenden Gruppe gewählt wird.

28. Assay nach einem der Ansprüche 25 bis 27, worin der Linker der Sonde von der Formel I ist.

29. Assay nach Anspruch 24, worin der Linker der Sonde von der Formel IX oder der Formel X ist, worin die Nukleinsäure der Sonde aus 20-150 Nukleotiden in der komplementären Sequenz zu der Sequenz des Zielsegments besteht; das katalytisch aktive Enzym eine eukaryotische Peroxidase, z.B. Meerrettichperoxidase, ist; und R₁ aus der aus einer Bindung zwischen den Stickstoffatomen, (NH)(CO)(NH) und (NH)(CO)(CH₂)ₙ(CO)(NH) bestehenden Gruppe gewählt wird, worin n 0 bis 10 ist.

30. Assay nach Anspruch 29, worin die Nukleinsäure der Sonde eine Lange von 25-50 Nukleotiden aufweist; und R₁ aus der aus einer Bindung zwischen den Stickstoffatomen, (NH)(CO)(NH) und (NH)(CO)(CH₂)₄(CO)(NH) bestehenden Gruppe gewählt wird.

31. Assay nach Anspruch 29 oder 30, worin der Linker der Sonde von der Formel IX ist.

32. Sonde nach einem der Ansprüche 1 bis 7, oder Nukleinsäure nach einem der Ansprüche 8 bis 10, oder Verfahren nach einem der Ansprüche 11 bis 23, oder Assay nach einem der Ansprüche 24 bis 31, worin die Nukleinsäure oder, im Falle des Assays, die Nukleinsäure der Sonde eine DNA ist.

33. Verwendung einer Sonde nach einem der Ansprüche 1 bis 7, oder einer Nukleinsäure nach einem der Ansprüche 8 bis 10 oder 32 bei einem Hybridisierungsassay bzw. bei der Herstellung einer Sonde.

## Revendications

1. Une sonde d'acide nucléique qui comprend un acide nucléique monocaténaire et une enzyme catalytiquement active, dans laquelle/
- ledit acide nucléique comprend un segment d'au moins 20 nucléotides dans la séquence complémentaire à la séquence du segment cible de ladite sonde;
- ledit acide nucléique et ladite enzyme sont liés par un élément de liaison dont la formule est choisie dans le groupe comprenant:
Formule I:
-(PO₃)NH(R₁)N=CH(R₂)(CO)- I
et Formule II:
-(PO₃)NH(R₁)NH(CH₂)(R₂)(CO)- II
formules dans lesquelles:
. R₁ est choisi dans le groupe comprenant: la liaison entre atomes d'azote, (NH)(CO)(R₁₁)(CO)(NH), (NH)(CO)(NH) et 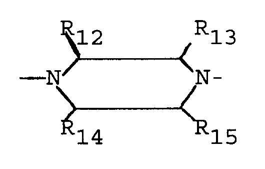 où:
R₁₁ est une liaison entre les atomes de carbone des groupes (CO) ou un radical alkylène comportant 1 à 10 atomes de carbone,
R₁₂, R₁₃, R₁₄ et R₁₅, identiques et/ou différents, sont choisis dans le groupe comprenant l'atome d'hydrogène et le radical alkyle en C₁ à C₃;
. R₂ est choisi dans le groupe comprenant les radicaux alkylènes en C₁ à C₂₀ et le radical arylène de formule: 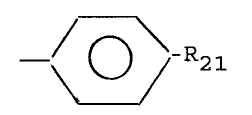 dans laquelle R₂₁ est un radical alkylène comportant de 0 à 10 atomes de carbone;
. -(PO₃)- est lié à l'atome de carbone 5' du nucléotide 5' de l'acide nucléique; et
. les atomes de carbone des groupes carbonyles situés du côté droit des formules I et II, sont liés à l'atome d'azote du groupe amino libre de l'enzyme.

2. Une sonde d'acide nucléique selon la revendication 1, dans laquelle:
- l'acide nucléique consiste en 20-150 nucléotides dans la séquence complémentaire à la séquence du segment cible;
- l'enzyme catalytiquement active est choisie dans le groupe comprenant phosphatases, peroxydases, béta-galactosidases, uréases, anhydrases carboniques et luciférases;
R₁ est choisi dans le groupe comprenant la liaison entre atomes d'azote; (NH)(CO)(NH) et (NH)(CO)(CH₂)ₙ(CO)(NH), où n est un nombre de 0 à 10; et
- R₂ est choisi dans le groupe comprenant le radical p-phénylène et (CH₂)m, où m est un nombre de 1 à 10.

3. Une sonde d'acide nucléique selon la revendication 2, dans laquelle:
l'acide nucléique présente une longueur de 25-50 nucléotides;
- R₁ est choisi dans le groupe comprenant la liaison entre atomes d'azote, (NH)(CO)(NH) et (NH)(CO)(CH₂)₄(CO)(NH), et
- R₂ est le radical p-phénylène.

4. Une sonde d'acide nucléique selon l'une quelconque des revendications 1 à 3, dans laquelle l'enzyme est choisie dans le groupe comprenant phosphatase alcaline d'intestin de veau, phosphatase alcaline de E. coli, peroxydase de raifort, béta-galactosidase de E. coli, uréase de fève de jaquier, anhydrase carbonique d'érythrocyte bovin, luciférase de P. fischeri et luciférase de luciole.

5. Une sonde d'acide nucléique qui comprend un acide nucléique monocaténaire et une enzyme catalytiquement active, dans laquelle:
- ledit acide nucléique comprend un segment d'au moins 20 nucléotides dans la séquence complémentaire à la séquence du segment cible de ladite sonde;
- ladite enzyme est une glycoprotéine qui a été oxydée par traitement avec du periodate;
- ledit acide nucléique et ladite enzyme sont liés par un élément de liaison présentant une formule choisie dans le groupe comprenant:
la formule IX:
-(PO₃)NH(R₁)N= IX
et la formule X:
-(PO₃)NH(R₁)NH- X
formules dans lesquelles:
. R₁ est choisi dans le groupe comprenant: la liaison entre atomes d'azote, (NH)(CO)(R₁₁)(CO)(NH), (NH)(CO)(NH) et 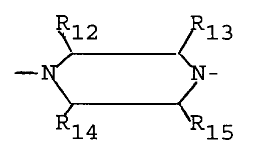 où:
R₁₁ est une liaison entre les atomes de carbone des groupes (CO) ou un radical alkylène comportant 1 à 10 atomes de carbone,
R₁₂, R₁₃, R₁₄ et R₁₅, identiques et/ou différents, sont choisis dans le groupe comprenant l'atome d'hydrogène et le radical alkyle en C₁ à C₃;
. -(PO₃)- est lié à l'atome de carbone 5' du nucléotide 5' de l'acide nuclèique;
- -N= qui est situé à droite dans la formule IX et l'atome d'azote du groupe -NH- situé à droite dans la formule X sont liés à un atome de carbone d'un résidu sucre de l'enzyme, qui correspond à l'atome de carbone du groupe carbonyle formé lors du traitement de l'enzyme avec du periodate.

6. Une sonde d'acide nucléique selon la revendication 5, dans laquelle:
- l'acide nucléique consiste en 20 à 150 nucléotides dans la séquence complémentaire à la séquence du segment cible;
- l'enzyme catalytiquement active est une peroxydase eucaryote, par exemple une peroxydase de raifort; et
- R₁ est choisi dans le groupe comprenant la liaison entre atomes d'azote, (NH)(CO)(NH) et (NH)(CO)(CH₂)ₙ(CO)(NH), dans laquelle n est un nombre compris entre 0 et 10.

7. Une sonde d'acide nucléique selon la revendication 5, dans laquelle l'acide nucléique présente une longueur de 25 à 50 nucléotides, et R₁ est choisi dans le groupe comprenant la liaison entre atomes d'azote, (NH)(CO)(NH) et (NH)(CO)(CH₂)₄(CO)(NH).

8. Un acide nucléique monocaténaire qui est un intermédiaire pour la préparation d'une sonde d'acide nucléique pour la détection d'un segment cible, comprenant un segment d'au moins 20 nucléotides dans la séquence complémentaire à celle dudit segment cible, et qui est substitué par un radical de formule XIV:
-(PO₃)(NH)(R₁)NH₂ XIV
dans laquelle:
. R₁ est choisi dans le groupe comprenant: la liaison entre atomes d'azote, (NH)(CO)(R₁₁)(CO)(NH), (NH)(CO)(NH) et 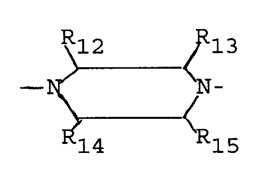 où:
R₁₁ est une liaison entre les atomes de carbone des groupes (CO) ou un radical alkylène comportant 1 à 10 atomes de carbone,
R₁₂, R₁₃, R₁₄ et R₁₅, identiques et/ou différents, sont choisis dans le groupe comprenant l'atome d'hydrogène et le radical alkyle en C₁ à C₃;
. -(PO₃)- est lié à l'atome de carbone 5' du nucléotide 5' de l'acide nucléique;

9. Un acide nucléique selon la revendication 8, dans lequel l'acide nucléique consiste en 20 à 150 nucléotides dans la séquence complémentaire à la séquence du segment cible; et R₁ est choisi dans le groupe comprenant la liaison entre atomes d'azote, (NH)(CO)(NH) et (NH)(CO)(CH₂)ₙ(CO)(NH), où n est un nombre compris entre 0 et 10.

10. Un acide nucléique selon la revendication 9, dans lequel l'acide nucléique présente une longueur de 25 à 50 nucléotides, et R₁ est choisi dans le groupe comprenant la liaison entre atomes d'azote, (NH)(CO)(NH) et (NH)(CO)(CH₂)₄(CO)(NH).

11. Un procédé de préparation d'une sonde d'acide nucléique qui comprend un acide nucléique monocaténaire et une enzyme catalytiquement active, dans lequel:
- ledit acide nucléique comprend un segment d'au moins 20 nucléotides dans la séquence complémentaire à la séquence du segment cible de ladite sonde;
- ledit acide nucléique et ladite enzyme sont liés par un élément de liaison de formule I:
-(PO₃)NH(R₁)N=CH(R₂)(CO)- I
formule dans laquelle:
. R₁ est choisi dans le groupe comprenant: la liaison entre atomes d'azote, (NH)(CO)(R₁₁)(CO)(NH), (NH)(CO)(NH) et 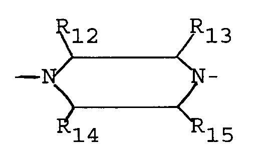 où:
R₁₁ est une liaison entre les atomes de carbone des groupes (CO) ou un radical alkylène comportant 1 à 10 atomes de carbone,
R₁₂, R₁₃, R₁₄ et R₁₅, identiques et/ou différents, sont choisis dans le groupe comprenant l'atome d'hydrogène, un radical alkyle en C₁ à C₃;
. R₂ est choisi dans le groupe comprenant les radicaux alkylènes en C₁ à C₂₀ et le radical arylène de formule: 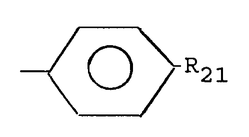 dans laquelle R₂₁ est un radical alkylène comportant de 0 à 10 atomes de carbone;
. -(PO₃)- est lié à l'atome de carbone 5' du nucléotide 5' de l'acide nucléique;
. les atomes de carbone du groupe carbonyle situé du côté droit de la formule I est lié à l'atome d'azote du groupe amino libre de l'enzyme,
lequel procédé comprend la réaction d'une solution aqueuse, tamponnée à un pH compris entre 7,0 et 9,0, d'un acide nucléique monocaténaire qui présente la même séquence que celle de l'acide nucléique de la sonde et est substitué par un radical de formule XIV:
-(PO₃)(NH)(R₁)NH₂ XIV
dans laquelle le groupe -(PO₃)- est lié à l'atome de carbone 5' du nucléotide 5' de l'acide nucléique,
avec une enzyme catalytiquement active, qui est substituée au niveau d'un groupe amino libre par un radical de formule XVIII:
(CHO)(R₂)(CO)- XVIII

12. Un procédé selon la revendication 11, dans lequel:
- l'acide nucléique consiste en 20-150 nucléotides dans la séquence complémentaire à la séquence du segment cible;
- l'enzyme catalytiquement active est choisie dans le groupe comprenant phosphatases, peroxydases, béta-galactosidases, uréases, anhydrases carboniques et luciférases;
- R₁ est choisi dans le groupe comprenant la liaison entre atomes d'azote; (NH)(CO)(NH) et (NH)(CO)(CH₂)ₙ(CO)(NH), où n est un nombre de 0 à 10; et
- R₂ est choisi dans le groupe comprenant le radical p-phénylène et (CH₂)m, où m est un nombre de 1 à 10.

13. Un procédé selon la revendication 12, dans lequel:
- l'acide nucléique présente une longueur de 25-50 nucléotides;
- R₁ est choisi dans le groupe comprenant la liaison entre atomes d'azote, (NH)(CO)(NH) et (NH)(CO)(CH₂)₄(CO)(NH), et
- R₂ est le radical p-phénylène.

14. Un procédé selon l'une quelconque des revendications 11 à 13, dans laquelle l'enzyme est choisie dans le groupe comprenant phosphatase alcaline d'intestin de veau, phosphatase alcaline de E. coli, peroxydase de raifort, béta-galactosidase de E. coli, uréase de fève de jaquier, anhydrase carbonique d'érythrocyte bovin, luciférase de P. fischeri et luciférase de luciole.

15. Un procédé de préparation d'une sonde d'acide nucléique qui comprend un acide nucléique monocaténaire et une enzyme catalytiquement active, dans lequel:
- ledit acide nucléique comprend un segment d'au moins 20 nucléotides dans la séquence complémentaire à la séquence du segment cible de ladite sonde;
- ladite enzyme est une glycoprotéine qui a été oxydée par traitement avec du periodate;
- ledit acide nucléique et ladite enzyme sont liés par une liaison de formule IX:
-(PO₃)NH(R₁)N= IX
formule dans laquelle:
. R₁ est choisi dans le groupe comprenant: la liaison entre atomes d'azote, (NH)(CO)(R₁₁)(CO)(NH), (NH)(CO)(NH) et 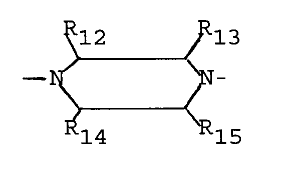 où:
R₁₁ est une liaison entre les atomes de carbone des groupes (CO) ou un radical alkylène comportant 1 à 10 atomes de carbone,
R₁₂, R₁₃, R₁₄ et R₁₅, identiques et/ou différents, sont choisis dans le groupe comprenant l'atome d'hydrogène et le radical alkyle en C₁ à C₃;
. -(PO₃)- est lié à l'atome de carbone 5' du nucléotide 5' de l'acide nucléique;
- -N= qui est situé à droite dans la formule IX et est lié à un atome de carbone d'un résidu sucre de l'enzyme, qui correspond à l'atome de carbone du groupe carbonyle formé lors du traitement de l'enzyme avec du periodate.
lequel procédé comprend la réaction dans une solution aqueuse, tamponnée à un pH compris entre 7,0 et 9,0, d'un acide nucléique monocaténaire, qui présente la même séquence que celle de la sonde d'acide nucléique est substitué par un radical représenté par la formule XIV:
-(PO₃)(NH)(R₁)NH₂ XIV
dans laquelle le groupe -(PO₃) est lié à l'atome de carbone 5' du nucléotide 5', avec ladite enzyme catalytiquement active.

16. Un procédé selon la revendication 15, dans lequel l'acide nucléique consiste en 20-150 nucléotides dans la séquence complémentaire à la séquence du segment cible de la sonde; l'enzyme catalytiquement active est une peroxydase eucaryote, par exemple une peroxydase de raifort; et R₁ est choisi dans le groupe comprenant la liaison entre atomes d'azote, (NH)(CO)(NH) et (NH)(CO)(CH₂)ₙ(CO)(NH), où n est un nombre de 0 à 10.

17. Un procédé selon la revendication 16, dans lequel l'acide nucléique présente une longueur de 25-50 nucléotides; et R₁ est choisi dans le groupe comprenant la liaison entre atomes d'azote, (NH)(CO)(NH) et (NH)(CO)(CH₂)₄(CO)(NH).

18. Un procédé de préparation d'une première sonde d'acide nucléique comprenant un acide nucléique monocaténaire et une enzyme catalytiquement active, dans lequel:
- ledit acide nucléique comprend un segment d'au moins 20 nucléotides dans la séquence complémentaire à la séquence du segment cible de ladite sonde;
- ledit acide nucléique et ladite enzyme sont liés par un premier élément de liaison: de formule II:
-(PO₃)NH(R)NH(CH₂)(R₂)(CO)- II
ou de formule XXIX:
-(PO₃)NH(R₁)NH- XXIX
formules dans lesquelles:
. R₁ est choisi dans le groupe comprenant: la liaison entre atomes d'azote, (NH)(CO)(R₁₁)(CO)(NH), (NH)(CO)(NH) et 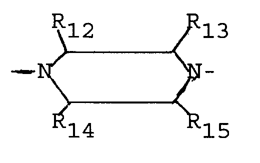 où:
R₁₁ est une liaison entre les atomes de carbone des groupes (CO) ou un radical alkylène comportant 1 à 10 atomes de carbone,
R₁₂, R₁₃, R₁₄ et R₁₅, identiques et/ou différents, sont choisis dans le groupe comprenant l'atome d'hydrogène et le radical alkyle en C₁ à C₃;
. R₂ est choisi dans le groupe comprenant les radicaux alkylènes en C₁ à C₂₀ et le radical arylène de formule: 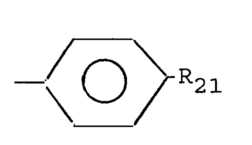 dans laquelle R₂₁ est un radical alkylène comportant de 0 à 10 atomes de carbone;
. -(PO₃)- est lié à l'atome de carbone 5' du nucléotide 5' de l'acide nucléique;
à condition que si la formule du premier élément de liaison est la formule II, le carbone du groupe -(CO)- situé à droite dans ladite formule soit lié à un groupe amino libre de l'enzyme;
et à condition que si la formule dudit premier élément de liaison est la formule XXIX, l'enzyme catalytiquement active soit une glycoprotéine et qu'elle ait été oxydée par du periodate, et que l'atome d'azote du groupe -NH- situé à droite dans ladite formule soit lié à un atome de carbone d'un résidu sucre de l'enzyme, qui était l'atome de carbone d'un groupe carbonyle formé au cours du traitement de l'enzyme avec du periodate;
lequel procédé comprend la réaction dans une solution aqueuse, tamponnée à un pH compris entre 5 et 9, d'un sel de métal alcalin de cyanoborohydrure avec une seconde sonde d'acide nucléique qui est sensiblement la même que ladite première sonde d'acide nucléique, sauf que, dans ladite seconde sonde, l'acide nucléique et l'enzyme sont liés par un second élément de liaison
de formule I:
-(PO₃)NH(R₁)N=CH(R₂)(CO)- I
ou de formule IX:
-(PO₃)NH(R₁)N= IX
à la place dudit premier élément de liaison, à condition que si ledit premier élément de liaison répond à la formule II, ledit second élément de liaison réponde à la formule I, et que si ledit premier élément de liaison répond à la formule XXIX, ledit second élément de liaison réponde à la formule IX.

19. Un procédé selon la revendication 18, dans lequel:
- le premier élément de liaison répond à la formule II,
- l'acide nucléique consiste en 20-150 nucléotides dans la séquence complémentaire à la séquence du segment cible,
- l'enzyme catalytiquement active est choisie dans le groupe comprenant phosphatases, peroxydases, béta-galactosidases, uréases, anhydrases carboniques et luciférases,
- R₁ est choisi dans le groupe comprenant la liaison entre atomes d'azote, (NH)(CO)(NH) et (NH)(CO)(CH₂)ₙ(CO)(NH), où n est un nombre de 0 à 10; et
- R₂ est choisi dans le groupe comprenant le radical p-phénylène et (CH₂)ₘ, où m est un nombre compris entre 1 et 10.

20. Un procédé selon la revendication 19, dans lequel:
- l'acide nucléique présente une longueur de 25-50 nucléotides;
- R₁ est choisi dans le groupe comprenant la liaison entre atomes d'azote, (NH)(CO)(NH) et (NH)(CO)(CH₂)₄(CO)(NH); et
- R₂ est le radical p-phénylène.

21. Un procédé selon l'une quelconque des revendications 18 à 20, dans lequel l'enzyme est choisie dans le groupe comprenant phosphatase alcaline d'intestin de veau, phosphatase alcaline de E. coli, peroxydase de raifort, béta-galactosidase de E. coli, uréase de fève de jaquier, anhydrase carbonique d'érythrocyte de bovin, luciférase de P. fischeri et luciférase de luciole.

22. Un procédé selon la revendication 18, dans lequel:
- le premier élément de liaison répond à la formule XXIX;
- l'acide nucléique consiste en 20-150 nucléotides dans la séquence complémentaire à la séquence du segment cible;
- l'enzyme catalytiquement active est une peroxydase eucaryote, par exemple l'hydroxydase de raifort; et
- R₁ est choisi dans le groupe comprenant la liaison entre atomes d'azote; (NH)(CO)(NH) et (NH)(CO)(CH₂)ₙ(CO)(NH), où n est un nombre de 0 à 10.

23. Un procédé selon la revendication 22, dans lequel l'acide nucléique présente une longueur de 25-50 nucléotides; et R₁ est choisi dans le groupe comprenant la liaison entre atomes d'azote, (NH)(CO)(NH) et (NH)(CO)(CH₂)₄(CO)(NH).

24. Un test d'hybridation d'une sonde d'acide nucléique pour un analyte d'acide nucléique, qui comprend la séquence cible d'au moins 20 nucléotides dans une séquence connue, ledit test comprenant l'utilisation en tant que sonde d'acide nucléique pour la détection de l'analyte d'acide à base d'acide nucléique dans ledit test, d'une sonde qui comprend un acide nucléique monocaténaire et une enzyme catalytiquement active, dans laquelle:
- l'acide nucléique de la sonde comprend un segment avec la séquence complémentaire de la séquence dudit segment cible;
- ledit acide nucléique et ladite enzyme sont liés par un élément de liaison présentant une formule choisie dans le groupe comprenant:
Formule I:
-(PO₃)NH(R₁)N=CH(R₂)(CO)- I
Formule II:
-(PO₃)NH(R₁)NH(CH₂)(R₂)(CO)- II
Formule IX:
-(PO₃)NH(R₁)N= IX
et Formule X:
-(PO₃)NH(R₁)NH- X
formules dans lesquelles:
. R₁ est choisi dans le groupe comprenant: la liaison entre atomes d'azote, (NH)(CO)(R₁₁)(CO)(NH), (NH)(CO)(NH) et 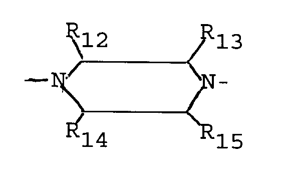 où :
R₁₁ est une liaison entre les atomes de carbone des groupes (CO) ou un radical alkylène comportant 1 à 10 atomes de carbone,
R₁₂, R₁₃, R₁₄ et R₁₅, identiques et/ou différents, sont choisis dans le groupe comprenant l'atome d'hydrogène et le radical alkyle en C₁ à C₃;
. R₂ est choisi dans le groupe comprenant les radicaux alkylènes en C₁ à C₂₀ et le radical arylène de formule: 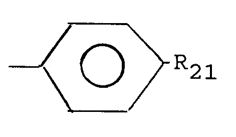 dans laquelle R₂₁ est un radical alkylène comportant de 0 à 10 atomes de carbone;
. -(PO₃)- est lié à l'atome de carbone 5' du nucléotide 5' de l'acide nucléique;
. les atomes de carbone des groupes carbonyles situés du côté droit des formules I et II, sont liés à l'atome d'azote du groupe amino libre de l'enzyme - à condition que si l'élément de liaison répond à la formule IX ou à la formule X, l'enzyme catalytiquement active soit une glycoprotéine qui a été oxydée par le periodate et -N= situé à droite dans la formule IX et l'atome d'azote du groupe -NH- situé à droite dans la formule X, sont liés à un atome de carbone du résidu sucre de l'enzyme, qui correspond à l'atome de carbone d'un groupe carbonyle formé lors du traitement de l'enzyme avec le periodate.

25. Un test selon la revendication 24, dans lequel:
- l'élément de liaison de la sonde présente la formule I ou la formule II,
- l'acide nucléique de la sonde consiste en 20-150 nucléotides dans la séquence complémentaire à la séquence du segment cible;
- l'enzyme catalytiquement active est choisie dans le groupe comprenant phosphatases, peroxydases, béta-galactosidases, uréases, anhydrases carboniques et luciférases;
- R₁ est choisi dans le groupe comprenant la liaison entre atomes d'azote; (NH)(CO)(NH) et (NH)(CO)(CH₂)ₙ(CO)(NH), où n est un nombre de 0 à 10; et
- R₂ est choisi dans le groupe comprenant le radical p-phénylène et (CH₂)m, dans lequel m est un nombre de 1 à 10.

26. Un test selon la revendication 25, dans lequel l'acide nucléique de la sonde présente une longueur de 25-50 nucléotides; R₁ est choisi dans le groupe comprenant la liaison entre atomes d'azote, (NH)(CO)(NH) et (NH)(CO)(CH₂)₄(CO)(NH), et R₂ est le radical p-phénylène.

27. Un test selon l'une quelconque des revendications 24 à 26, dans lequel l'enzyme de la sonde est choisie dans le groupe comprenant phosphatase alcaline d'intestin de veau, phosphatase alcaline de E. coli, peroxydase de raifort, béta-galactosidase de E. coli, uréase de fève de jaquier, anhydrase carbonique d'érythrocyte bovin, luciférase de P. fischeri et luciférase de luciole.

28. Un test selon l'une quelconque des revendications 25 à 27, dans lequel l'élément de liaison de la sonde présente la formule I.

29. Un test selon la revendication 24, dans lequel:
- l'élément de liaison de la sonde présente la formule IX ou la formule X,
- l'acide nucléique de la sonde consiste en 20-150 nucléotides dans la séquence complémentaire à la séquence du segment cible;
- l'enzyme catalytiquement active est une peroxydase eucaryote, par exemple une peroxydase de raifort; et
- R₁ est choisi dans le groupe comprenant la liaison entre atomes d'azote; (NH)(CO)(NH) et (NH)(CO)(CH₂)ₙ(CO)(NH), où n est un nombre de 0 à 10.

30. Un test selon la revendication 29, dans lequel l'acide nucléique de la sonde présente une longueur de 25-50 nucléotides; et R₁ est choisi dans le groupe comprenant la liaison entre atomes d'azote, (NH)(CO)(NH) et (NH)(CO)(CH₂)₄(CO)(NH).

31. Un test selon la revendication 29 ou la revendication 30, dans lequel l'élément de liaison de la sonde est représenté par la formule IX.

32. Une sonde selon l'une quelconque des revendications 1 à 7, ou un acide nucléique selon l'une quelconque des revendications 8 à 10, ou un procédé selon l'une quelconque des revendications 11 à 23, ou un test selon l'une quelconque des revendications 24 à 31, dans lesquels l'acide nucléique ou, dans le cas dudit test l'acide nucléique de la sonde, est un ADN.

33. Utilisation d'une sonde selon l'une quelconque des revendications 1 à 7 ou d'un acide nucléique selon l'une quelconque des revendications 8 à 10 ou 32 dans un test d'hybridation ou pour la préparation d'une sonde, respectivement.
